# EUROPEAN PATENT SPECIFICATION

(11) **EP 1 749 892 B1**
(45) Date of publication and mention of the grant of the patent: **19.03.2008**
(21) Application number: 06015952.2
(22) Date of filing: 01.08.2006
(51) Int. Cl.: C12Q 1/68

(54) **Nucleotide sequence for assessing TSE**
Nukleotidsequenz zur Detektion von TSE
Séquence nucléotidique permettant d'évaluer TSE

(30) Priority: 02.08.2005 EP 05016740; 26.08.2005 EP 05018546
(43) Date of publication of application: 07.02.2007
(73) Proprietor: Roche Diagnostics GmbH, 68305 Mannheim (DE); F. HOFFMANN-LA ROCHE AG, 4070 Basel (CH)
(72) Inventor: Knoll, Michael, 82377 Penzberg (DE); Eberle, Walter, 82347 Bernried (DE); Seitz, Christoph, 82481 Mittenwald (DE)

(56) References cited:
- WO-A-20/05049863
- DE-A1- 19 918 141
- DATABASE EMBL 18 December 2003 (2003-12-18), "Bovine immunodeficiency virus provirus gene SEQ.ID.NO.1" XP002361426 Database accession no. ADC21643 & WO 03/066810 A (NOVARTIS AG; LUO, TIANCI; KALEKO, MICHAEL; GOLIGHTLY, DOUGLAS; MOLINA,) 14 August 2003 (2003-08-14)
- DATABASE EMBL 29 July 2003 (2003-07-29), XP002361418 Database accession no. AC145779
- DATABASE EMBL 11 February 2005 (2005-02-11), XP002361419 Database accession no. AC157263
- XIANG WEI ET AL: "Identification of differentially expressed genes in scrapie-infected mouse brains by using global gene expression technology" JOURNAL OF VIROLOGY, vol. 78, no. 20, October 2004 (2004-10), pages 11051-11060, XP002361333 ISSN: 0022-538X
- RIEMER C ET AL: "Gene expression profiling of scrapie-infected brain tissue" BIOCHEMICAL AND BIOPHYSICAL RESEARCH COMMUNICATIONS, ACADEMIC PRESS INC. ORLANDO, FL, US, vol. 323, no. 2, 15 October 2004 (2004-10-15), pages 556-564, XP004562763 ISSN: 0006-291X
- BROWN A R ET AL: "Identification of up-regulated genes by array analysis in scrapie-infected mouse brains" NEUROPATHOLOGY AND APPLIED NEUROBIOLOGY, vol. 30, no. 5, October 2004 (2004-10), pages 555-567, XP002361334 ISSN: 0305-1846
- LU ZHI YUN ET AL: "New molecular markers of early and progressive CJD brain infection" JOURNAL OF CELLULAR BIOCHEMISTRY, LISS, NEW YORK, NY, US, vol. 93, no. 4, 1 November 2004 (2004-11-01), pages 644-652, XP002352431 ISSN: 0730-2312
- BAKER C A ET AL: "Unique inflammatory RNA profiles of microglia in Creutzfeldt-Jakob disease" PROCEEDINGS OF THE NATIONAL ACADEMY OF SCIENCES OF USA, NATIONAL ACADEMY OF SCIENCE, WASHINGTON, DC, US, vol. 100, no. 2, 21 January 2003 (2003-01-21), pages 675-679, XP002353413 ISSN: 0027-8424
- BAKER CHRISTOPHER A ET AL: "Microglia from Creutzfeldt-Jakob disease-infected brains are infectious and show specific mRNA activation profiles" JOURNAL OF VIROLOGY, vol. 76, no. 21, November 2002 (2002-11), pages 10905-10913, XP002361335 ISSN: 0022-538X
- RIEMER CONSTANZE ET AL: "Identification of upregulated genes in scrapie-infected brain tissue" JOURNAL OF VIROLOGY, vol. 74, no. 21, November 2000 (2000-11), pages 10245-10248, XP002361336 ISSN: 0022-538X
- DANDOY-DRON FRANCOISE ET AL: "Enhanced levels of scrapie responsive gene mRNA in BSE-infected mouse brain" MOLECULAR BRAIN RESEARCH, vol. 76, no. 1, 10 March 2000 (2000-03-10), pages 173-179, XP002361337 ISSN: 0169-328X
- BAKER C A ET AL: "MICROGLIAL ACTIVATION VARIES IN DIFFERENT MODELS OF CREUTZFELDT-JAKOB DISEASE" JOURNAL OF VIROLOGY, THE AMERICAN SOCIETY FOR MICROBIOLOGY, US, vol. 73, no. 6, June 1999 (1999-06), pages 5089-5097, XP008036207 ISSN: 0022-538X
- DANDOY-DRON R ET AL: "GENE EXPRESSION IN SCRAPIE CLONING OF A NEW SCRAPIE-RESPONSIVE GENE AND THE IDENTIFICATION OF INCREASED LEVELS OF SEVEN OTHER MRNA TRANSCRIPTS" JOURNAL OF BIOLOGICAL CHEMISTRY, AMERICAN SOCIETY OF BIOLOCHEMICAL BIOLOGISTS, BIRMINGHAM,, US, vol. 273, no. 13, 27 March 1998 (1998-03-27), pages 7691-7697, XP000941399 ISSN: 0021-9258
- DIEDRICH J ET AL: "IDENTIFYING AND MAPPING CHANGES IN GENE EXPRESSION INVOLVED IN THE NEUROPATHOLOGY OF SCRAPIE AND ALZHEIMER'S DISEASE" CURRENT TOPICS IN MICROBIOLOGY AND IMMUNOLOGY, SPRINGER, BERLIN,, DE, vol. 172, 1991, pages 259-274, XP009020897 ISSN: 0070-217X
- LUHR KATARINA M ET AL: "Cathepsin B and L are involved in degradation of prions in GTI-1 neuronal cells" NEUROREPORT, vol. 15, no. 10, 19 July 2004 (2004-07-19), pages 1663-1667, XP009058727 ISSN: 0959-4965
- LUHR KATARINA M ET AL: "Scrapie protein degradation by cysteine proteases in CD11c+ dendritic cells and GT1-1 neuronal cells" JOURNAL OF VIROLOGY, vol. 78, no. 9, May 2004 (2004-05), pages 4776-4782, XP002361338 ISSN: 0022-538X
- ZHANG YONGHUA ET AL: "Up-regulation of cathepsin B and cathepsin L activities in scrapie-infected mouse Neuro2a cells." JOURNAL OF GENERAL VIROLOGY, vol. 84, no. 8, August 2003 (2003-08), pages 2279-2283, XP002361339 ISSN: 0022-1317
- DOH-URA KATSUMI ET AL: "Lysosomotropic agents and cysteine protease inhibitors inhibit scrapie-associated prion protein accumulation" JOURNAL OF VIROLOGY, vol. 74, no. 10, May 2000 (2000-05), pages 4894-4897, XP002361340 ISSN: 0022-538X
- DIEDRICH J F ET AL: "NEUROPATHOLOGICAL CHANGES IN SCRAPIE AND ALZHEIMER'S DISEASE ARE ASSOCIATED WITH INCREASED EXPRESSION OF APOLIPOPROTEIN E AND CATHEPSIN D IN ASTROCYTES" JOURNAL OF VIROLOGY, NEW YORK, US, US, vol. 65, no. 9, 1 September 1991 (1991-09-01), pages 4759-4768, XP000443989 ISSN: 0022-538X
- SUPATTAPONE SURACHAI ET AL: "Pharmacological approaches to prion research" BIOCHEMICAL PHARMACOLOGY, vol. 63, no. 8, 15 April 2002 (2002-04-15), pages 1383-1388, XP002361346 ISSN: 0006-2952
- DATABASE EMBL 3 April 2004 (2004-04-03), XP002361427 Database accession no. CL509257
- DATABASE EMBL 20 November 2003 (2003-11-20), XP002361428 Database accession no. CG855679
- DATABASE EMBL 6 June 2003 (2003-06-06), XP002361429 Database accession no. CD495179

### SEQUENCE LISTING

<110> KNOLL, MICHAEL
   EBERLE, WALTER
   SEITZ, CHRISTOPH
<120> NUCLEOTIDE SEQUENCE FOR ASSESSING TSE
<130> 23255 US
<140>
   <141>
<150> EP 05018546.1
   <151> 2005-08-26
<150> EP 05016740.2
   <151> 2005-08-02
<160> 27
<170> PatentIn Ver. 3.3
<210> 1
   <211> 369
   <212> DNA
   <213> Bos taurus
<400> 1
   agcgtgtggc tggagggcga cgtcattttg tcccctgtgt ggacttgtgt gaccagcccc 60
   gccctggaat actgaactgt cccagcacct cccgtgcatc agttgtgaga tctcggagca 120
   gtgtgctggg ccaggctggg cagctgatcc aacccctggc cctgcggtgg gtgttggggt 180
   ttttcccgct ctggagccgt taccagcaca gctgctgtga agggctgcgt tggtattgga 240
   cgtcgagtgt ataatcacat gacctctcgt cgctcaggac ggacgcccag gggtctggtc 300
   gccccgtgag ctcacgtctc ctttccttct tgcatctcga tcagcgatgt gtgatggagt 360
   cctcggtgg                                                         369
<210> 2
   <211> 123
   <212> PRT
   <213> Bos taurus
<220>
   <221> MOD_RES
   <222> (32)
   <223> Variable amino acid
<400> 2
   Pro Pro Arg Thr Pro Ser His Ile Ala Asp Arg Asp Ala Arg Arg Lys
   1 5 10 15
   Gly Asp Val Ser Ser Arg Gly Asp Gln Thr Pro Gly Arg Pro Ser Xaa
   20 25 30
   Ala Thr Arg Gly His Val Ile Ile His Ser Thr Ser Asn Thr Asn Ala
   35 40 45
   Ala Leu His Ser Ser Cys Ala Gly Asn Gly Ser Arg Ala Gly Lys Thr
   50 55 60
   Pro Thr Pro Thr Ala Gly Pro Gly Val Gly Ser Ala Ala Gln Pro Gly
   65 70 75 80
   Pro Ala His Cys Ser Glu Ile Ser Gln Leu Met His Gly Arg Cys Trp
   85 90 95
   Asp Ser Ser Val Phe Gln Gly Gly Ala Gly His Thr Ser Pro His Arg
   100 105 110
   Gly Gln Asn Asp Val Ala Leu Gln Pro His Ala
   115 120
<210> 3
   <211> 26
   <212> DNA
   <213> Artificial Sequence
<220>
   <223> Description of Artificial Sequence: Synthetic primer
<400> 3
   tctcaaaaca ggcctctgcc cggtgg 26
<210> 4
   <211> 26
   <212> DNA
   <213> Artificial Sequence
<220>
   <223> Description of Artificial Sequence: Synthetic primer
<400> 4
   tgaggggtca ggagatccca gcgaca 26
<210> 5
   <211> 17
   <212> DNA
   <213> Artificial Sequence
<220>
   <223> Description of Artificial Sequence: Synthetic primer
<400> 5
   cctcccgtgc atcagtt 17
<210> 6
   <211> 17
   <212> DNA
   <213> Artificial Sequence
<220>
   <223> Description of Artificial Sequence: Synthetic primer
<400> 6
   tgagcgacga gaggtca 17
<210> 7
   <211> 17
   <212> DNA
   <213> Artificial Sequence
<220>
   <223> Description of Artificial Sequence: Synthetic primer
<400> 7
   ccgctctgga gccgtta 17
<210> 8
   <211> 23
   <212> DNA
   <213> Artificial Sequence
<220>
   <223> Description of Artificial Sequence: Synthetic primer
<400> 8
   tgatcgagat gcaagaagga aag 23
<210> 9
   <211> 17
   <212> DNA
   <213> Artificial Sequence
<220>
   <223> Description of Artificial Sequence: Synthetic primer
<400> 9
   tgggcagctg atccaac 17
<210> 10
   <211> 23
   <212> DNA
   <213> Artificial Sequence
<220>
   <223> Description of Artificial Sequence: Synthetic primer
<400> 10
   gagatgcaag aaggaaagga gac 23
<210> 11
   <211> 17
   <212> DNA
   <213> Artificial Sequence
<220>
   <223> Description of Artificial Sequence: Synthetic primer
<400> 11
   gaagggctgc gttggta 17
<210> 12
   <211> 18
   <212> DNA
   <213> Artificial Sequence
<220>
   <223> Description of Artificial Sequence: Synthetic primer
<400> 12
   caccgaggac tccatcac 18
<210> 13
   <211> 18
   <212> DNA
   <213> Artificial Sequence
<220>
   <223> Description of Artificial Sequence: Synthetic primer
<400> 13
   gagggcgacg tcattttg 18
<210> 14
   <211> 17
   <212> DNA
   <213> Artificial Sequence
<220>
   <223> Description of Artificial Sequence: Synthetic primer
<400> 14
   tgagcgacga gaggtca 17
<210> 15
   <211> 23
   <212> DNA
   <213> Artificial Sequence
<220>
   <223> Description of Artificial Sequence: Synthetic primer
<400> 15
   ctgatcgaga tgcaagaagg aaa 23
<210> 16
   <211> 14
   <212> DNA
   <213> Artificial Sequence
<220>
   <223> Description of Artificial Sequence: Synthetic primer
<400> 16
   gctcacgggg cgac 14
<210> 17
   <211> 189
   <212> DNA
   <213> Bos taurus
<400> 17
   cctcccgtgc atcagttgtg agatctcgga gcagtgtgct gggccaggct gggcagctga 60
   tccaacccct ggccctgcgg tgggtgttgg ggtttttccc gctctggagc cgttaccagc 120
   acagctgctg tgaagggctg cgttggtatt ggacgtcgag tgtataatca catgacctct 180
   cgtcgctca                                                         189
<210> 18
   <211> 334
   <212> PRT
   <213> Homo sapiens
<400> 18
   Met Pro Ile Asn Arg Met Trp Gly Leu Thr Val Leu Leu Leu Pro Val
    1               5                  10                  15
   Val Ser Phe Ala Leu Tyr Pro Glu Glu Ile Leu Asp Thr Gln Trp Glu
               20                  25                  30
   Leu Trp Lys Lys Thr Tyr Arg Lys Gln Tyr Asn Ser Lys Gly Asp Glu
           35                  40                  45
   Ile Ser Arg Arg Leu Ile Trp Glu Lys Asn Leu Lys His Ile Ser Ile
       50                  55                  60
   His Asn Leu Glu Ala Ser Leu Gly Val His Thr Tyr Glu Leu Ala Met
   65                  70                  75                  80
   Asn His Leu Gly Asp Met Thr Ser Glu Glu Val Val Gln Lys Met Thr
                   85                  90                  95
   Gly Leu Lys Val Pro Ala Ser Arg Ser Arg Ser Asn Asp Thr Leu Tyr
               100                 105                 110
   Ile Pro Asp Trp Glu Gly Arg Ala Pro Asp Ser Val Asp Tyr Arg Lys
           115                 120                 125
   Lys Gly Tyr Val Thr Pro Val Lys Asn Gln Gly Gln Cys Gly Ser Cys
       130                 135                 140
   Trp Ala Phe Ser Ser Val Gly Ala Leu Glu Gly Gln Leu Lys Lys Lys
   145                 150                 155                 160
   Thr Gly Lys Leu Leu Asn Leu Ser Pro Gln Asn Leu Val Asp Cys Val
                   165                 170                 175
   Ser Glu Asn Asp Gly Cys Gly Gly Gly Tyr Met Thr Asn Ala Phe Gln
               180                 185                 190
   Tyr Val Gln Lys Asn Arg Gly Ile Asp Ser Glu Asp Ala Tyr Pro Tyr
           195                 200                 205
   Val Gly Gln Asp Glu Asn Cys Met Tyr Asn Pro Thr Gly Lys Ala Ala
       210                 215                 220
   Lys Cys Arg Gly Tyr Arg Glu Ile Pro Glu Gly Asn Glu Lys Ala Leu
   225                 230                 235                 240
   Lys Arg Ala Val Ala Arg Val Gly Pro Ile Ser Val Ala Ile Asp Ala
                   245                 250                 255
   Ser Leu Thr Ser Phe Gln Phe Tyr Arg Lys Gly Val Tyr Tyr Asp Glu
               260                 265                 270
   Asn Cys Asn Ser Asp Asn Leu Asn His Ala Val Leu Ala Val Gly Tyr
           275                 280                 285
   Gly Ile Gln Lys Gly Asn Lys His Trp Ile Ile Lys Asn Ser Trp Gly
   290 295 300
   Glu Asn Trp Gly Asn Lys Gly Tyr Ile Leu Met Ala Arg Asn Lys Asn
   305 310 315 320
   Asn Ala Cys Gly Ile Ala Asn Leu Ala Ser Phe Pro Lys Met
   325 330
<210> 19
   <211> 334
   <212> PRT
   <213> Bos taurus
<400> 19
   Met Pro Ile Asn Arg Met Trp Gly Leu Thr Val Leu Leu Leu Pro Val
   1 5 10 15
   Val Ser Phe Ala Leu Tyr Pro Glu Glu Ile Leu Asp Thr Gln Trp Glu
   20 25 30
   Leu Trp Lys Lys Thr Tyr Arg Lys Gln Tyr Asn Ser Lys Gly Asp Glu
   35 40 45
   Ile Ser Arg Arg Leu Ile Trp Glu Lys Asn Leu Lys His Ile Ser Ile
   50 55 60
   His Asn Leu Glu Ala Ser Leu Gly Val His Thr Tyr Glu Leu Ala Met
   65 70 75 80
   Asn His Leu Gly Asp Met Thr Ser Glu Glu Val Val Gln Lys Met Thr
   85 90 95
   Gly Leu Lys Val Pro Ala Ser Arg Ser Arg Ser Asn Asp Thr Leu Tyr
   100 105 110
   Ile Pro Asp Trp Glu Gly Arg Ala Pro Asp Ser Val Asp Tyr Arg Lys
   115 120 125
   Lys Gly Tyr Val Thr Pro Val Lys Asn Gln Gly Gln Cys Gly Ser Cys
   130 135 140
   Trp Ala Phe Ser Ser Val Gly Ala Leu Glu Gly Gln Leu Lys Lys Lys
   145 150 155 160
   Thr Gly Lys Leu Leu Asn Leu Ser Pro Gln Asn Leu Val Asp Cys Val
   165 170 175
   Ser Glu Asn Asp Gly Cys Gly Gly Gly Tyr Met Thr Asn Ala Phe Gln
   180 185 190
   Tyr Val Gln Lys Asn Arg Gly Ile Asp Ser Glu Asp Ala Tyr Pro Tyr
   195 200 205
   Val Gly Gln Asp Glu Asn Cys Met Tyr Asn Pro Thr Gly Lys Ala Ala
   210 215 220
   Lys Cys Arg Gly Tyr Arg Glu Ile Pro Glu Gly Asn Glu Lys Ala Leu
   225 230 235 240
   Lys Arg Ala Val Ala Arg Val Gly Pro Ile Ser Val Ala Ile Asp Ala
   245 250 255
   Ser Leu Thr Ser Phe Gln Phe Tyr Arg Lys Gly Val Tyr Tyr Asp Glu
   260 265 270
   Asn Cys Asn Ser Asp Asn Leu Asn His Ala Val Leu Ala Val Gly Tyr
   275 280 285
   Gly Ile Gln Lys Gly Asn Lys His Trp Ile Ile Lys Asn Ser Trp Gly
   290 295 300
   Glu Asn Trp Gly Asn Lys Gly Tyr Ile Leu Met Ala Arg Asn Lys Asn
   305 310 315 320
   Asn Ala Cys Gly Ile Ala Asn Leu Ala Ser Phe Pro Lys Met
   325 330
<210> 20
   <211> 329
   <212> PRT
   <213> Homo sapiens
<400> 20
<210> 21
   <211> 321
   <212> PRT
   <213> Homo sapiens
<400> 21
   Met Asp Val Arg Ala Leu Pro Trp Leu Pro Trp Leu Leu Trp Leu Leu
   1 5 10 15
   Cys Arg Gly Gly Gly Asp Ala Asp Ser Arg Ala Pro Phe Thr Pro Thr
   20 25 30
   Trp Pro Arg Ser Arg Glu Arg Glu Ala Ala Ala Phe Arg Glu Ser Leu
   35 40 45
   Asn Arg His Arg Tyr Leu Asn Ser Leu Phe Pro Ser Glu Asn Ser Thr
   50 55 60
   Ala Phe Tyr Gly Ile Asn Gln Phe Ser Tyr Leu Phe Pro Glu Glu Phe
   65 70 75 80
   Lys Ala Ile Tyr Leu Arg Ser Lys Pro Ser Lys Phe Pro Arg Tyr Ser
   85 90 95
   Ala Glu Val His Met Ser Ile Pro Asn Val Ser Leu Pro Leu Arg Phe
   100 105 110
   Asp Trp Arg Asp Lys Gln Val Val Thr Gln Val Arg Asn Gln Gln Met
   115 120 125
   Cys Gly Gly Cys Trp Ala Phe Ser Val Val Gly Ala Val Glu Ser Ala
   130 135 140
   Tyr Ala Ile Lys Gly Lys Pro Leu Glu Asp Leu Ser Val Gln Gln Val
   145 150 155 160
   Ile Asp Cys Ser Tyr Asn Asn Tyr Gly Cys Asn Gly Gly Ser Thr Leu
   165 170 175
   Asn Ala Leu Asn Trp Leu Asn Lys Met Gln Val Lys Leu Val Lys Asp
   180 185 190
   Ser Glu Tyr Pro Phe Lys Ala Gln Asn Gly Leu Cys His Tyr Phe Ser
   195 200 205
   Gly Ser His Ser Gly Phe Ser Ile Lys Gly Tyr Ser Ala Tyr Asp Phe
   210 215 220
   Ser Asp Gln Glu Asp Glu Met Ala Lys Ala Leu Leu Thr Phe Gly Pro
   225 230 235 240
   Leu Val Val Ile Val Asp Ala Val Ser Trp Gln Asp Tyr Leu Gly Gly
   245 250 255
   Ile Ile Gln His His Cys Ser Ser Gly Glu Ala Asn His Ala Val Leu
   260 265 270
   Ile Thr Gly Phe Asp Lys Thr Gly Ser Thr Pro Tyr Trp Ile Val Arg
   275 280 285
   Asn Ser Trp Gly Ser Ser Trp Gly Val Asp Gly Tyr Ala His Val Lys
   290 295 300
   Met Gly Ser Asn Val Cys Gly Ile Ala Asp Ser Val Ser Ser Ile Phe
   305 310 315 320
   Val
<210> 22
   <211> 321
   <212> PRT
   <213> Homo sapiens
<400> 22
   Met Asp Val Arg Ala Leu Pro Trp Leu Pro Trp Leu Leu Trp Leu Leu
   1 5 10 15
   Cys Arg Gly Gly Gly Asp Ala Asp Ser Arg Ala Pro Phe Thr Pro Thr
   20 25 30
   Trp Pro Arg Ser Arg Glu Arg Glu Ala Ala Ala Phe Arg Glu Ser Leu
   35 40 45
   Asn Arg His Arg Tyr Leu Asn Ser Leu Phe Pro Ser Glu Asn Ser Thr
   50 55 60
   Ala Phe Tyr Gly Ile Asn Gln Phe Ser Tyr Leu Phe Pro Glu Glu Phe
   65 70 75 80
   Lys Ala Ile Tyr Leu Arg Ser Lys Pro Ser Lys Phe Pro Arg Tyr Ser
   85 90 95
   Ala Glu Val His Met Ser Ile Pro Asn Val Ser Leu Pro Leu Arg Phe
   100 105 110
   Asp Trp Arg Asp Lys Gln Val Val Thr Gln Val Arg Asn Gln Gln Met
   115 120 125
   Cys Gly Gly Cys Trp Ala Phe Ser Val Val Gly Ala Val Glu Ser Ala
   130 135 140
   Tyr Ala Ile Lys Gly Lys Pro Leu Glu Asp Leu Ser Val Gln Gln Val
   145 150 155 160
   Ile Asp Cys Ser Tyr Asn Asn Tyr Gly Cys Asn Gly Gly Ser Thr Leu
   165 170 175
   Asn Ala Leu Asn Trp Leu Asn Lys Met Gln Val Lys Leu Val Lys Asp
   180 185 190
   Ser Glu Tyr Pro Phe Lys Ala Gln Asn Gly Leu Cys His Tyr Phe Ser
   195 200 205
   Gly Ser His Ser Gly Phe Ser Ile Lys Gly Tyr Ser Ala Tyr Asp Phe
   210 215 220
   Ser Asp Gln Glu Asp Glu Met Ala Lys Ala Leu Leu Thr Phe Gly Pro
   225 230 235 240
   Leu Val Val Ile Val Asp Ala Val Ser Trp Gln Asp Tyr Leu Gly Gly
   245 250 255
   Ile Ile Gln His His Cys Ser Ser Gly Glu Ala Asn His Ala Val Leu
   260 265 270
   Ile Thr Gly Phe Asp Lys Thr Gly Ser Thr Pro Tyr Trp Ile Val Arg
   275 280 285
   Asn Ser Trp Gly Ser Ser Trp Gly Val Asp Gly Tyr Ala His Val Lys
   290 295 300
   Met Gly Ser Asn Val Cys Gly Ile Ala Asp Ser Val Ser Ser Ile Phe
   305 310 315 320
   Val
<210> 23
   <211> 312
   <212> PRT
   <213> Mus musculus
<400> 23
   Met Lys Pro Gln Leu Val Asn Leu Leu Leu Leu Cys Cys Cys Cys Leu
   1 5 10 15
   Gly Arg His Gly Val Ala Gly Thr Trp Ser Trp Ser His Gln Arg Glu
   20 25 30
   Ala Ala Ala Leu Arg Glu Ser Leu His Arg His Arg Tyr Leu Asn Ser
   35 40 45
   Phe Pro His Glu Asn Ser Thr Ala Phe Tyr Gly Val Asn Gln Phe Ser
   50 55 60
   Tyr Leu Phe Pro Glu Glu Phe Lys Ala Leu Tyr Leu Gly Ser Lys Tyr
   65 70 75 80
   Ala Trp Ala Pro Arg Tyr Pro Ala Glu Gly Gln Arg Pro Ile Pro Asn
   85 90 95
   Val Ser Leu Pro Leu Arg Phe Asp Trp Arg Asp Lys His Val Val Asn
   100 105 110
   Pro Val Arg Asn Gln Glu Met Cys Gly Gly Cys Trp Ala Phe Ser Val
   115 120 125
   Val Ser Ala Ile Glu Ser Ala Arg Ala Ile Gln Gly Lys Ser Leu Asp
   130 135 140
   Tyr Leu Ser Val Gln Gln Val Ile Asp Cys Ser Phe Asn Asn Ser Gly
   145 150 155 160
   Cys Leu Gly Gly Ser Pro Leu Cys Ala Leu Arg Trp Leu Asn Glu Thr
   165 170 175
   Gln Leu Lys Leu Val Ala Asp Ser Gln Tyr Pro Phe Lys Ala Val Asn
   180 185 190
   Gly Gln Cys Arg His Phe Pro Gln Ser Gln Ala Gly Val Ser Val Lys
   195 200 205
   Asp Phe Ser Ala Tyr Asn Phe Arg Gly Gln Glu Asp Glu Met Ala Arg
   210 215 220
   Ala Leu Leu Ser Phe Gly Pro Leu Val Val Ile Val Asp Ala Met Ser
   225 230 235 240
   Trp Gln Asp Tyr Leu Gly Gly Ile Ile Gln His His Cys Ser Ser Gly
   245 250 255
   Glu Ala Asn His Ala Val Leu Ile Thr Gly Phe Asp Arg Thr Gly Asn
   260 265 270
   Thr Pro Tyr Trp Met Val Arg Asn Ser Trp Gly Ser Ser Trp Gly Val
   275 280 285
   Glu Gly Tyr Ala His Val Lys Met Gly Gly Asn Val Cys Gly Ile Ala
   290 295 300
   Asp Ser Val Ala Ala Val Phe Val
   305 310
<210> 24
   <211> 22
   <212> PRT
   <213> Artificial Sequence
<220>
   <223> Description of Artificial Sequence: Synthetic Cys motif
<400> 24
   Cys Ser Glu Ile Ser Gln Leu Met His Gly Arg Cys Trp Asp Ser Ser
   1 5 10 15
   Val Phe Gln Gly Gly Ala
   20
<210> 25
   <211> 57
   <212> DNA
   <213> Bos taurus
<400> 25
   gatgcacggg aggtgctggg acagttcagt attccagggc ggggctggtc acacaag 57
<210> 26
   <211> 46
   <212> DNA
   <213> Artificial Sequence
<220>
   <223> Description of Artificial Sequence: Synthetic oligonucleotide
<400> 26
   gatgcacggg aggtgctggg acagttcagt attccagggc ggggct 46
<210> 27
   <211> 60
   <212> DNA
   <213> Bos taurus
<400> 27
   aacgcttgtg gcattgccaa cctggccagc ttccccaaga tgtgacttcc accagccaac 60

## Description

The present invention is directed to the assessment of TSE using a sample from a living individual. The assessment is based on the use of an RNA marker molecule in a sample of whole blood. The invention is further directed to the detection of the marker molecule by means of real-time PCR.

### Background of the Invention

Transmissible spongiform encephalopathies (TSEs) consist of a unique group of invariably fatal neurological disorders which affect both human and animals. TSEs are characterized by long presymptomatic incubation periods of months or years. Brain lesions associated with deposits of protease-resistant proteins are a hallmark of clinically manifest TSE disease.

Variant Creutzfeldt-Jakob disease (vCJD) is a rare and fatal human neurodegenerative condition. The classical form, that is to say CJD, presents as a subacute dementia, evolving over weeks to several months and is accompanied by pyramidal, extrapyramidal, and cerebellar signs. The mean age of death is 57 years but the disease may occur in the late teens and early twenties. In the final stages of the disease, there is an incapacitating dementia, usually with severe myoclonus. Recently, a variant of CJD (vCJD) was described in UK and France with distinct clinical and pathological features which affected younger individuals (average age 29 years, as opposed to 65 years).

As with CJD, vCJD is classified as a TSE because of characteristic spongy degeneration of the brain and its ability to be transmitted. The presumed infectious agent of TSE is often termed PrP^{sc} or PrP^{res} denoting the disease-specific form of the prion protein (PrP). The biological properties of PrP^{sc} in vCJD and BSE share common biological properties e.g. they have similar incubation periods in various kinds of mice and hamsters. TSEs are also known in other animals. For instance, scrapie affects sheep and goats and has been found in many sheep-producing countries throughout the world for over 250 years. Chronic Wasting Disease (CWD) is a contagious fatal TSE in cervids (members of the deer and elk family).

The hypothesis of a link between vCJD and BSE was first raised because of the association of these two TSEs in time and place. More recent evidence supporting a link including identification of pathological features similar to vCJD in brains of macaque monkeys inoculated with BSE. A vCJD-BSE link is further supported by the demonstration that vCJD is associated with a molecular marker that distinguishes it from other forms of CJD and which resembles that seen in BSE transmitted to a number of other species. Studies of the distribution of the infectious agent in the brains of mice artificially infected with tissues from humans with vCJD and cows with BSE showed nearly identical patterns. The most recent and powerful evidence comes from studies showing that the transmission characteristics of BSE and vCJD in laboratory mice are almost identical, strongly indicating that they are due to the same causative agent. In conclusion, the most likely cause of vCJD is exposure to the BSE agent, most plausibly due to dietary contamination by affected bovine central nervous system tissue.

It follows that with regard to the human food chain there is an urgent need for methods to assess a possible TSE infection in the living host. Particularly there is a desire to assess TSE in living animals at a pre-symptomatic stage and before the animals are slaughtered and processed to enter the human food chain. One way to assess TSE is commonly applied when testing slaughtered cattle routinely for bovine spongiform encephalopathy. A brain sample is derived from a sacrificed animal and the presence of PrP^{sc} in the sample is assessed by means of an immunological test such as an ELISA or a Western blot. An alternative approach targets TSE disease specific changes of gene expression. Neurodegeneration in the brain is apparently closely linked with alterations within the members of the cellular defense system of the central nervous system. Notably, increased expression of distinct genes encoding cathepsin enzymes in microglia and/or astrocytes was found to be a correlate of spongiform encephalopathies.

Microglia and astrocytes are both glial cells. Glial cells are the connective tissue cells of the central nervous system (CNS), serving as the supportive structure that holds together and protects neurons. Astrocytes form one particular type of glial cell. They are relatively large with thread-like projections (star shape) that connect with neurons and small blood vessels (capillaries). These projections form part of the blood-brain barrier. This barrier slows or prevents the passage of unwanted substances, such as harmful chemicals, infectious agents, etc., from the bloodstream into the brain. Astrocytes also accumulate in areas where nerves have been damaged (astrocytosis), sealing off these areas. Microglial cells account for approx. 20% of the total glial population in the central nervous system. They are distributed with no significant local differences in the white and gray matters. In contrast to astrocytes they cover non-overlapping territories. They belong to the mononuclear phagocyte system and form the resident macrophages in the brain tissue, the spinal cord and the retina. Their function in the normal neural parenchyma is unknown. However, in various pathologies they form a most reactive sensor to threats to the nervous system and upon stimulation within a few hours they exhibit an activation program. Microglia are highly reactive, mobile and multifunctional immune cells of the CNS that can play a universal role in the defense of the neural parenchyma. Activated microglial cells become immuno-competent and brain macrophages of microglial origin possess cathepsins B and L which render them potentially cytotoxic (Kreutzberg, G.W., Arzneimittelforschung 45 (1995) 357-360).

Intracellular proteinases such as members of the cathepsin families play a role in the process of neuronal cell death. Apparently, this is also the case during neurodegenerative diseases. An increase in cathepsins B, L, and D (lysosomal proteinases) and cathepsin E (non-lysosomal aspartic proteinase) in neurons has been shown in the early stage of neuronal degeneration. An increased level of such proteinases was also found in reactive glial cells and may be involved in the pathogenesis of neurodegenerative disease (Nakanishi, H., and Yamamoto, K., Japanese Journal of Pharmacology 105 (1995) 1-9 [abstract]).

With respect to TSE neurodegeneration, Diedrich, J.F., et al., J. Virol 65 (1991) 4759-4768 report that neuropathological changes in scrapie are associated with a higher level of cathepsin D mRNA. Increased expression was demonstrated by analysis of mRNA isolates from brains of mice experimentally infected with scrapie 16 weeks postinoculation. Furthermore, overexpression of the mRNAs was mapped to astrocytes. Using an experimental scrapie mouse model and cDNA expression arrays together with quantitative RT-PCR, Brown, A.R., et al., Neuropathology and Applied Neurobiology 30 (2004) 555-567 showed increased expression of cathepsin D in hippocampal tissue. Using microarray analysis of gene expression in mouse brains after infection with two different strains of scrapie Xiang, W., et al., J. Virology 78 (2004) 11051-11060 showed significant up-regulation for cathepsins S, H, C, D, and Z. To a much lesser, if not insignificant extent the expression of cathepsins B and L appeared to be higher in infected brains. In an experimental mouse model for CJD expression of the gene encoding cathepsin S was found to be induced significantly in microglia cells of the brain (Baker, C.A., et al., J. Virology 73 (1999) 5089-5097). An increase by the factor of about 2 was measured between 88 and 120 days after inoculation. In a later report by Baker, C.A., et al., J. Virology 76 (2002) 10905-10913 it is disclosed that steady-state levels of mRNA for cathepsin S were increased about 10-fold in CJD-infected microglia which were isolated from mouse brains. In a further study Baker, C.A., and Manuelidis, L., PNAS 100 (2003) 675-679 found enhanced transcription of the genes encoding cathepsins S, L, and H in microglia isolated from CJD-infected mouse brains. However, another study of CJD infected mouse brains by Lu, Z.Y., et al., J. Cellular Biochemistry 93 (2004) 644-652 found that the expression of cathepsin D and, conflicting with the previously cited disclosure, cathepsin L was not altered over the entire course of infection. Dandoy-Dron, F., J. Biol. Chem. 273 (1998) 7691-7697 describe an analysis by means of mRNA differential display of mouse brains infected with scrapie. It was found that, among other transcripts, the cathepsin S mRNA was preferentially expressed giving rise to a higher abundance of the transcript compared to the uninfected controls. When mice were inoculated intracerebrally with the infectious agent that causes BSE, the abundance of cathepsin S mRNA in mouse brain was enhanced likewise.

So far, the state of the art only presents data about increased transcription of cathepsin genes in brain cells, as a result of TSE. Also, the studies of the state of the art regarding marker molecules are confined to artificial disease models in experimental settings. Particularly, in these models the species barrier is crossed. E.g., mice are infected with the scrapie agent from sheep or the vCJD agent from man.

When serving as a marker for the assessment of TSE, cathepsin gene expression is used in the analysis of a brain sample or even a sampled subpopulation of brain cells (microglia). Using whole brain or specific brain cells as sample material, however, necessitates extensive work and safety precautions while preparing the sample material. E.g., the skull of the animal needs to be opened manually and all objects that come in contact with the brain material are at risk of getting contaminated with contagious material.

### Summary of the Invention

The object of the present invention was to provide a new marker molecule as a means for assessing a TSE infection which can be analyzed in a sample other than brain material. Preferably, the sample can be taken before an individual suspected to suffer from TSE has died or, in the case of an animal, is killed.

Surprisingly the inventors found that the presence of a nucleotide sequence encoding a fragment of a hypothetical cystein proteinase according to SEQ ID NO:2 in whole blood correlates with TSE infection. Thus, according to the present invention the problem is solved by the use of a nucleotide sequence encoding a fragment of a hypothetical cystein proteinase according to SEQ ID NO:2 or a nucleotide sequence complementary thereto, as a marker in the assessment of transmissible spongiform encephalopathy (TSE) in a bovine. Further, the invention provides a method for assessing transmissible spongiform encephalopathy (TSE) in a bovine by detecting a target RNA, comprising the consecutive steps, in a sample obtained from the bovine, of (a) preparing RNA from said sample, (b) detecting the target RNA in the RNA preparation of step (a), (c) using the result of step (b) to assess TSE in the bovine, characterized in that the sample from the bovine is whole blood, and the target RNA encodes an amino acid sequence according to SEQ ID NO:2 or a subfragment thereof comprising 6 or more consecutive amino acids. In addition, the invention provides kits of parts for assessing TSE in a mammal by detecting a target RNA encoding a fragment of the hypothetical cystein proteinase according to SEQ ID NO:2 or a subfragment thereof according to appending claims 10-13.

### Detailed Description of the Invention

As a consequence of TSE progression the expression of a number of genes is changed in certain cell types. Detection of such changes can be used for diagnosing TSE, thereby offering an alternative to immunological assays for prion protein (PrP^{sc}). The inventors have surprisingly found in cattle that the presence of an RNA sequence according to SEQ ID NO: 1 in whole blood correlates with TSE infection.

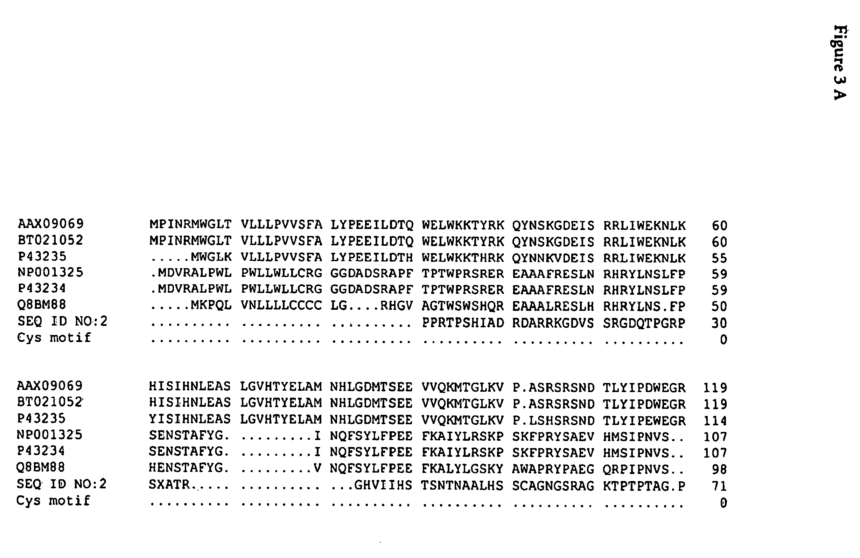

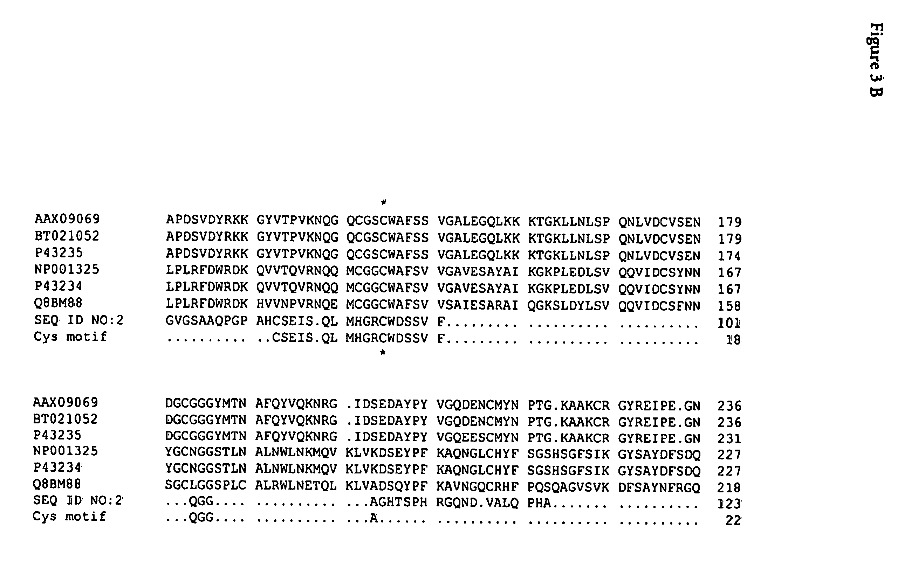

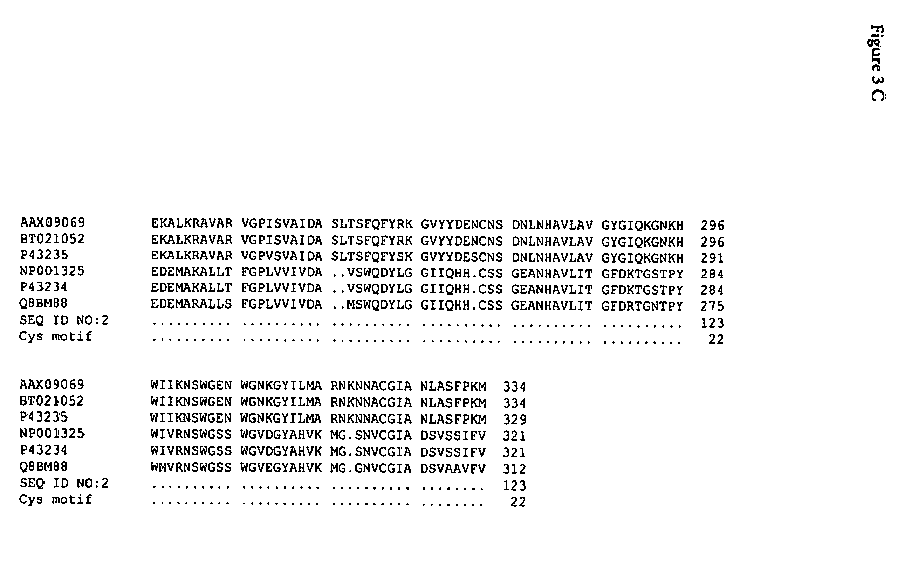

Initial screening of total nucleic acids from blood samples revealed a pair of PCR primers according to SEQ ID NO:3 and SEQ ID NO: 4 which allowed to amplify a target nucleic acid with a size of about 400 bp. The fragment was found only in samples from cattle which were infected with BSE but could not be detected in uninfected animals. Sequencing revealed the nucleotide sequence of SEQ ID NO: 1 comprising 369 bp. The amplified target nucleic acid of the band at approximately 400 bp was verified to be identical in all positive samples where it turned up. A computer-aided search for the largest uninterrupted coding frame revealed an amino acid sequence of 91 residues as given in SEQ ID NO:2. A homology search indicated that this sequence is a fragment of a novel hypothetical cystein proteinase. On the level of amino acid sequence the fragment according to SEQ ID NO:2 shows some homology to murine cathepsin O precursor (swissprot accession Q8BM88), human cathepsin O precursor (swissprot accession P43234; Protein database at the National Center for Biotechnology Information (NCBI), accession No. NP_001325), human cathepsin K precursor (swissprot accession P43235), and bovine cathepsin K precursor (encoded by the mRNA according to NCBI Nucleotide accession BT021052; NCBI Protein accession AAX09069). An alignment of the amino acid sequences is shown in Figure 3. Particularly, the cysteine residue typical for the catalytic center of cysteine proteases appears to be conserved supporting the hypothesis that SEQ ID NO:2 is a fragment of a cysteine proteinase. Nevertheless, the amino acid sequence of SEQ ID NO:2 appears to be unique, so far.

Apparently, the mRNA encoding the hypothetical cystein proteinase is a correlate of TSE in cattle. A first embodiment of the invention is therefore the use of a nucleotide sequence encoding a fragment of a hypothetical cystein proteinase according to SEQ ID NO:2 or a nucleotide sequence complementary thereto, as a marker in the assessment of transmissible spongiform encephalopathy (TSE) in a bovine.

According to the invention it is preferred that a nucleotide sequence encoding a subfragment of SEQ ID NO:2 comprising 6 or more amino acids is used as a marker for TSE. More preferred is the use of the nucleotide sequence according to SEQ ID NO: 1 or a nucleotide sequence complementary thereto, as a marker in the assessment of transmissible spongiform encephalopathy (TSE). Even more preferred, the nucleotide sequence is used as a marker to assess TSE in a bovine. Even more preferred, the nucleotide sequence has a size of about 150-200 bp.

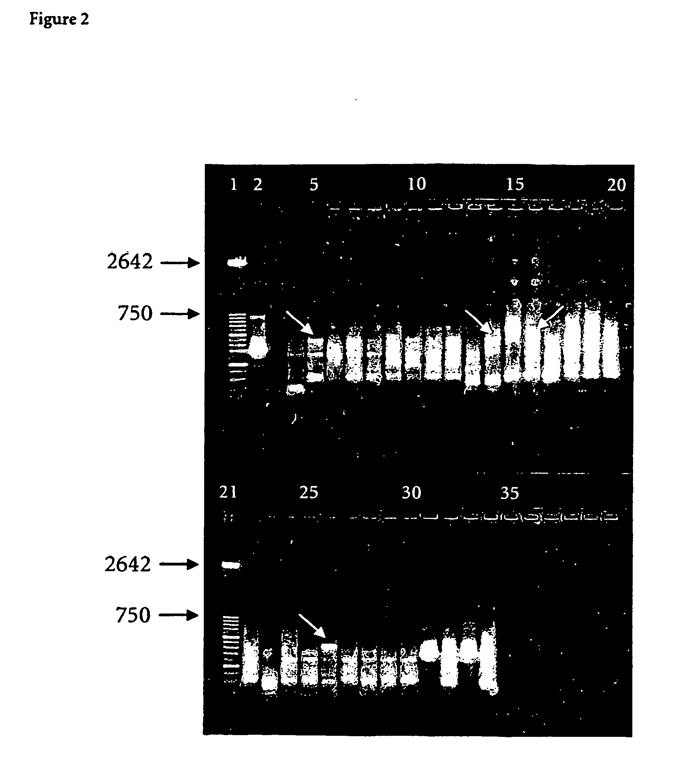

A further embodiment of the invention is a method for assessing TSE in a bovine by detecting a target RNA, comprising the consecutive steps, in a sample obtained from the bovine, (a) preparing RNA from said sample, (b) detecting the target RNA in the RNA preparation of step (a), (c) using the result of step (b) to assess TSE in the mammal, characterized in that the sample from the bovine is whole blood, and the target RNA encodes an amino acid sequence according to SEQ ID NO:2 or a subfragment thereof comprising 6 or more amino acids. Most preferred, the subfragment is the sequence of SEQ ID NO:17. As shown in Figure 2, the target RNA of the primer pair according to SEQ ID NOs: 3 and 4 is undetectable in all samples of whole blood from TSE-free, i.e. uninfected individuals. In contrast, the target RNA is detectable in individuals suspected of suffering from TSE (TSE suspects) or individuals already showing clear signs of TSE. It is emphasized that the assessment of TSE according to the invention does not depend on post mortem samples such as, e.g., brain samples. In contrast, the invention provides the means to assess TSE using whole blood samples which can be obtained with great ease from living individuals.

With great advantage the present invention is used for assessing TSE in cattle. Therefore, in a further preferred embodiment of the invention the whole blood sample is taken from a living bovine animal. Even more preferred, the whole blood sample is obtained from an animal which is to proceed to the abattoir and to be processed for the human food chain later on. In this regard, the method of the invention can also be performed in combination with an immunological test detecting infectious prion protein (PrP^{sc}) or protease-resistant prion protein (PrP^{res}).

Before detection of the target RNA the fraction encompassing all nucleic acids (DNA and RNA) is purified from the sample which is a complex mixture of different components. Often, for the first steps, processes are used which allow the enrichment of the nucleic acids. To release the contents of cells, they may be treated with enzymes or with chemicals to dissolve, degrade or denature the cellular walls. This process is commonly referred to as lysis. The resulting solution containing such lysed material is referred to as lysate. A problem often encountered during the lysis is that other enzymes degrading the component of interest, e.g. ribonucleases degrading RNA, come into contact with the component of interest during lysis. These degrading enzymes may also be present outside the cells or may have been spatially separated in different cellular compartiments before the lysis and come now into contact with the component of interest. It is common to use chaotropic agents as e.g. guanidinium thiocyanate or anionic, cationic, zwitterionic or nonionic detergents when nucleic acids are intended to be set free. It is also an advantage to use proteases which rapidly degrade these enzymes or unwanted proteins. However, this may produce another problem as the said substances or enzymes can interfere with reagents or components in subsequent steps. Proteases (see Walsh, Enzymatic Reaction Mechanisms. W. H. Freeman and Company, San Francisco, Chapter 3 (1979)) which are commonly known to the are e.g. alkaline proteases (WO 98/04730) or acid proteases (US 5,386,024). The protease which is widely used in the prior art for sample preparation for the isolation of nucleic acids is proteinase K from Tritirachium album (see e.g. Sambrook, J. et al., Molecular Cloning: A Laboratory Manual, Cold Spring Harbor Laboratory Press, Cold Spring Harbor, New York, 1989) which is active around neutral pH and belongs to a family of proteases known as subtilisins.

In the next steps of the sample preparation which follow on the lysis step, the nucleic acids are further enriched. There are several methods for the extraction of nucleic acids: (A) sequence-dependent or biospecific methods such as e.g.: affinity chromatography and hybridisation to immobilised probes; (B) sequence-independent or physico-chemical methods such as e.g.: liquid-liquid extraction (e.g. with phenol-chloroform), precipitation (e.g. with pure ethanol), extraction with filter paper, extraction with micelle-forming agents (e.g. cetyl-trimethylammonium-bromide), binding to immobilised intercalating dyes (e.g. acridine derivatives), adsorption to silica gel or diatomic earths, and adsorption to magnetic glass particles (MGP) or organo silane particles under chaotropic conditions. Particularly interesting for extraction purposes is the adsorption of nucleic acids to a glass surface although other surfaces are possible. Many procedures for isolating nucleic acids from their natural environment have been proposed in recent years by the use of their binding behavior to glass surfaces. If unmodified nucleic acids are the target, a direct binding of the nucleic acids to a material with a silica surface or glass is preferred because among other reasons the nucleic acids do not have to be modified and even native nucleic acids can be bound. To separate the particles from the contaminants, the particles may be either centrifuged or fluids are drawn through glass fiber filters. This is a limiting step, however, that prevents the procedure from being used to process large quantities of samples. The use of magnetic particles to immobilize nucleic acids after precipitation by adding salt and ethanol is more advantageous and described e.g. in Alderton, R. P., et al., Anal. Biochem. 201 (1992) 166-169 and WO 91/12079. In this procedure, the nucleic acids are agglutinated along with the magnetic particles. The agglutinate is separated from the original solvent by applying a magnetic field and performing washing steps. After these washing steps, the nucleic acids are dissolved in a Tris buffer. Magnetizable particular adsorbents proved to be very efficient and suitable for automatic sample preparation. Ferrimagnetic and ferromagnetic as well as superparamagnetic pigments are used for this purpose. The most preferred magnetic glass particles and methods using these are described in WO 01/37291.

After the purification or isolation of the nucleic acids including the target nucleic acid from their natural surroundings, the target nucleic acid may be detected. Before detection, however, in an additional step the nucleic acids are incubated with RNase-free DNase, thereby allowing to specifically purify the RNAs which were present in the sample. Example 2 describes a preferred method of sample preparation using the MagNA Pure^{®} LC instrument and an RNA isolation kit (Roche Diagnostics GmbH, Mannheim, Germany).

It is particularly advantageous to detect the target RNA by way of specific amplification using RT-PCR (RT = reverse transcriptase; PCR = polymerase chain reaction). The target RNA is first reverse-transcribed to form a complementary single-stranded cDNA. The cDNA in turn serves as a template for DNA amplification using suitable primers. The amplification product is also referred to as the "target nucleic acid".

Accordingly, a very much preferred embodiment of the invention is a method comprising, in a whole blood sample obtained from the bovine; (a) preparing RNA from said sample; (b) providing a pair of primers comprising a first and a second primer, whereby the first primer consists of 12 or more contiguous nucleotides of a nucleic acid sequence encoding the amino acid sequence according to SEQ ID NO:2, and whereby the second primer consists of 12 or more contiguous nucleotides of a nucleic acid sequence complementary to the nucleic acid sequence encoding the amino acid sequence according to SEQ ID NO:2; (c) amplifying a target nucleic acid from the RNA preparation of step (a) with the primers of step (b); (d) detecting the target nucleic acid of step (c); (e) subsequently using the result of the detection of step (d) to assess TSE in the bovine. It is even more preferred that the target nucleic acid is amplified by way of polymerase chain reaction. With great advantage, detection of the target RNA of the invention is therefore achieved using RT-PCR. This method of amplifying a target nucleic acid is also well known to the skilled person. During RT-PCR the target RNA strand is "reverse" transcribed into its DNA complement, followed by amplification of the resulting DNA by means of PCR. Transcribing an RNA strand into its DNA complement is termed reverse transcription (RT), and is accomplished through the use of an RNA-dependent DNA polymerase (reverse transcriptase). Afterwards, a second strand of DNA is synthesized through the use of a deoxyoligonucleotide primer and a DNA-dependent DNA polymerase. The complementary DNA and its anti-sense counterpart are then exponentially amplified. The exponential amplification via RT-PCR provides for a highly sensitive technique, where very low copy number RNAs can be detected.

In a preferred embodiment of the invention the target nucleic acid has the nucleotide sequence of SEQ ID NO: 1 or is a fragment thereof. In the method of the invention a primer pair consisting of a first "forward" primer and a second "reverse" primer is used to amplify SEQ ID NO:1 or a fragment thereof as well as variants of said nucleotide sequence or fragments thereof. The first and the second primer are oligonucleotides with a preferred length of between 12 and 30 nucleotides, more preferred between 15 and 25 nucleotides. Preferably, the sequence of the first primer is a contiguous partial sequence of the nucleotide sequence of SEQ ID NO:1. Also preferred, the sequence of the second primer is a contiguous partial sequence of the complement of the nucleotide sequence of SEQ ID NO:1. Although there are numerous possibilities to design such primers, there are preferred primer sequences and combinations of primers according to the invention. For amplification the first primer is preferably selected from the group consisting of SEQ ID NO:3, SEQ ID NO:5, SEQ ID NO:7, SEQ ID NO:9, SEQ ID NO:11, and SEQ ID NO:13; the second primer is preferably selected from the group consisting of SEQ ID NO:4, SEQ ID NO:6, SEQ ID NO:8, SEQ ID NO:10, SEQ ID NO:12, SEQ ID NO:14, SEQ ID NO:15, and SEQ ID NO:16. It is most preferred that the first primer is the nucleotide sequence according to SEQ ID NO:5, and the second primer is the nucleotide sequence according to nucleotide sequence of SEQ ID NO:6.

It is noted that the terms "oligonucleotide" and also "polynucleotide" in the context of the present invention summarizes not only (desoxy)-oligo-ribonucleotides, but also all DNA- or RNA-derivatives known in the art like e.g. methyl-phosphonates, phosphothioates, 2'-O-alkyl-derivatives as well as peptide nucleic acids, and analoga comprising modified bases like 7-Deaza-Purines. It is also noted that a primer oligo- or polynucleotide is understood as being capable of serving as a substrate for template-dependent DNA- or RNA-polymerases. By virtue of polymerase activity a primer can be elongated by the addition of nucleoside phosphates or analogues thereof.

In the amplification reaction mixture the preferred initial concentration of each primer is preferably 0.5 µM.

Detection of the amplified target nucleic acid is possible by several means. In a preferred embodiment of the invention, the amplified target nucleic acid is detected using a fluorescent signal. Several detection formats based on target nucleic acid dependent fluorescent signaling have been disclosed, which enable continuous monitoring of the generation of amplification products (reviewed in Wittwer, et al., Biotechniques, 22, (1997) 130-138). These detection formats include but are not limited to (1) to (4):
(1) Use of fluorescent double-stranded DNA recognizing compounds: Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Preferably, only those dyes may be used which like SYBR^{®} Green I (Molecular Probes), for example, do not affect the efficiency of the PCR reaction. In a very much preferred embodiment of the invention, the target nucleic acid is detected by monitoring the amplification in real time and determining the amount of amplification product after each cycle. The fluorescent signal generated by the incorporated dye can be quantified. Signal strength correlates with the amount of PCR product formed and thus allows quantification of the target nucleic acid after each circle. Therefore, in an even more preferred embodiment of the invention the target nucleic acid is detected by monitoring the amplification in real time and determining the amount of amplification product after each cycle.
(2) Increased fluorescence resonance energy transfer (FRET) upon hybridization: For this detection format, two oligonucleotide hybridization probes each labeled with a fluorescent moiety are used which are capable of hybridizing to adjacent but non overlapping regions of one strand of the amplification product. Preferably, one oligonucleotide is labeled at the 5' end and the second oligonucleotide is labeled at the 3' end. When hybridized to the target DNA, the two fluorescent labels are brought into close contact, such that fluorescence resonance energy transfer between the two fluorescent moieties can take place. As a consequence, the hybridization can be monitored through excitation of the donor moiety and subsequent measurement of fluorescence emission of the second acceptor moiety. In a similar embodiment, only one fluorescently labeled probe is used, which together with one appropriately labeled primer may also serve as a specific FRET pair (Bernard, et al., Anal. Biochem. 255 (1998) 101-107). In a very much preferred embodiment of the invention, the target nucleic acid is detected with FRET hybridization probes. Independent from the detection format or fluorescent label, hybridization probes are always polynucleotides having sequences which are completely identical with or exactly complementary to the sequence of the target nucleic acid. Yet, it is also within the scope of the invention if the probes contain one or several mismatches, as long as they are capable of hybridizing to the amplification product under appropriate hybridization conditions. In any case, it has been proven to be particular advantageous, if the sequence identity or complementarity is 100% over a range of at least 10 contiguous residues. Taking onto account the length of the amplified fragments in the method of the invention, the length of the probe does not exceed 40 nucleotides, preferably not more than 30 nucleotides. However, hybridization probes may have 5' or 3' overhangs which do not hybridize to the target nucleic acid.
(3) Hydrolysis probes used in TaqMan^{®} instruments: In order to detect the amplification product, a single-stranded hybridization probe is used, which is labeled with a fluorescent entity, the fluorescence emission of which is quenched by a second label on the same probe which may act as a quenching compound. During the annealing step of the PCR reaction, the probe hybridizes to its target sequence, and, subsequently, during the extension of the primer, the DNA polymerase having a 5'-3'-exonuclease activity hydrolyzes the hybridization probe, such that the fluorescent entity is separated from the quencher compound. After appropriate excitation, fluorescence emission can be monitored as an indicator of accumulating amplification product.
(4) Molecular beacons: Similar to hydrolysis probes, a molecular beacon oligonucleotide is labeled with a fluorescent compound and a quencher compound, which due to the secondary structure of the molecule are in dose vicinity to each other. Upon binding to the target DNA, the intramolecular hydrogen bonding is broken, and the fluorescent compound located at one end of the probe is separated from the quencher compound, which is located at the opposite end of the probe (Lizardi et al., US 5, 118,801).

Hybridization probes such as hydrolysis probes or molecular beacons may be used for detection. Most preferred, however, are FRET hybridization probes, i.e. a pair of adjacently hybridizing probes, wherein upon hybridization the two fluorescent moieties are brought into close vicinity such that fluorescent resonance energy transfer (FRET) can take place. The term "FRET hybridization probes" therefore is defined as a pair of hybridization probes, each probe carrying a fluorescent compound, which together may act as a FRET pair thus enabling the detection of a nucleic acid, when both probes are hybridized adjacently to a target molecule.

With great advantage, the assay of the invention can be performed on a LightCycler^{®} instrument (Roche Diagnostics GmbH, Mannheim, Germany) using a pair of FRET hybridization probes labeled with Fluorescein at the 3' end of the first hybridization probe and with LightCycler^{®} Red 640 (Roche Diagnostics GmbH, Mannheim, Germany) at the 5' end of the second hybridization probe.

The skilled person is familiar with the design of hybridization probes for the detection of a target nucleic acid during real time PCR. There is also a wealth of auxiliary means for this purpose such as computer software. An example therefor is Roche LightCycler^{®} Probe Design Software 2.0 (Roche Diagnostics GmbH, Mannheim; catalogue no. 04 342 054 001) for the design of HybProbe^{®} and SimpleProbe^{®} hybridization probes.

Principally, any kind of quantification method can be applied, however, it has been proven to be advantageous, if methods using an external standard are applied. The external standard itself may be a plasmid or a linearized template with one or more target sequences to be amplified.

In case of quantification of a nucleic acid using external standards, a calibration curve has to be generated. For this calibration curve, known amounts of the target nucleic acid are amplified and the intensity of fluorescent signal is determined as a function of cycle number. After smoothening of the kinetics by a mathematical fit, the first or second maximum of the derivative are calculated. This enables a correlation between the original target concentration and the fractional cycle number of a determined maximum. Subsequently, determination of unknown analyte concentrations may be performed.

In order to eliminate quantification errors originating from different detection sensitivities, it has been proven to be particular advantageous if the same batch of hybridization probe(s) is used for the sample to be analyzed and for the calibration samples.

In addition, kits of parts are contemplated to facilitate practicing the invention. A further embodiment of the invention is therefore a kit of parts for assessing TSE in a mammal by detecting a target RNA, comprising (i) two oligonucleotide primers for amplification of a target nucleic acid as defined in appending claim 10, and (ii) an intercalating dye. A SYBR® Green dye is very much preferred. Yet, a further embodiment of the invention is a kit of parts for assessing TSE in a mammal by detecting a target RNA, comprising (i) two oligonucleotide primers for amplification of a target nucleic acid as defined in appending claim 11, and (ii) a first and a second oligonucleotide hybridization probe labeled with a first and a second fluorescent moiety, respectively, whereby the two oligonucleotide hybridization probes are complementary to adjacent but non overlapping regions of one strand of the target nucleic acid, whereby the first oligonucleotide hybridization probe is labeled at the 5' end and the second oligonucleotide hybridization probe is labeled at the 3' end, and whereby the first fluorescent labels are capable of effecting fluorescence resonance energy transfer. Yet, a further embodiment of the invention is a kit of parts for assessing TSE in a mammal by detecting a target RNA, comprising (i) two oligonucleotide primers for amplification of a target nucleic acid as defined in appending claim 12, and (ii) a single-stranded hybridization probe which is labeled with a fluorescent entity, the fluorescence emission of which is quenched by a second label on the same probe, whereby the single-stranded hybridization probe is complementary to a region of one strand of the target nucleic acid. Yet, a further embodiment of the invention is a kit of parts for assessing TSE in a mammal by detecting a target RNA, comprising (i) two oligonucleotide primers for amplification of a target nucleic acid as defined in appending claim 13, and (ii) a molecular beacon oligonucleotide complementary to a region of one strand of the target nucleic acid, whereby the molecular beacon oligonucleotide is labeled at opposite ends with a fluorescent compound and a quencher compound, which due to the secondary structure of said molecular beacon oligonucleotide are in dose vicinity to each other.

The kits of the invention preferably further comprise a polymerase enzyme with RNA-dependent DNA polymerase (reverse transcriptase) activity, a polymerase enzyme with DNA-dependent DNA polymerase activity, nucleotide triphosphates, buffers, a pair of primers comprising a first and a second primer, whereby the first primer consists of 12 or more contiguous nucleotides of a nucleic acid sequence encoding the amino acid sequence according to SEQ ID NO:2, and whereby the second primer consists of 12 or more contiguous nucleotides of a nucleic acid sequence complementary to the nucleic acid sequence encoding the amino acid sequence according to SEQ ID NO:2, a detection probe or sequence-unspecific detection means (intercalating dye, e.g. SYBR Green) and a positive control nucleic acid. The first primer is selected from the group consisting of SEQ ID NO:5, SEQ ID NO:7, SEQ ID NO:9, SEQ ID NO:11, and SEQ ID NO:13; the second primer is preferably selected from the group consisting of SEQ ID NO:6, SEQ ID NO:8, SEQ ID NO:10, SEQ ID NO:12, SEQ ID NO:14, SEQ ID NO:15, and SEQ ID NO:16. It is most preferred that the first primer is the nucleotide sequence according to SEQ ID NO:5, and the second primer is the nucleotide sequence according to nucleotide sequence of SEQ ID NO:6.

The following examples, sequence listing and figures are provided to aid the understanding of the present invention, the true scope of which is set forth in the appended claims. It is understood that modifications can be made in the procedures set forth without departing from the spirit of the invention.

### Description of the Figures

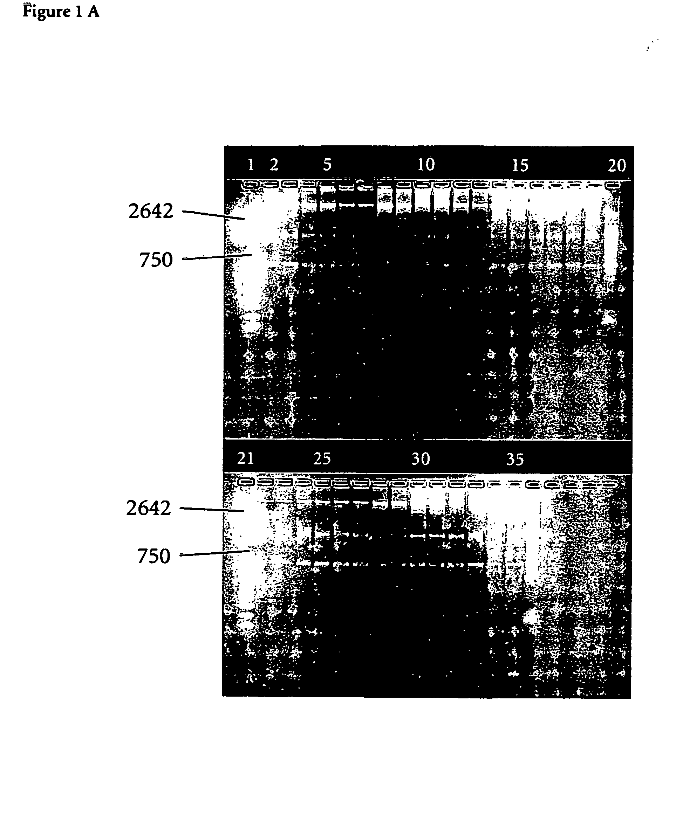

- **Figure 1 A**: Lanes 1 and 21: Size marker, fragment sizes in base pairs are indicated on the left. Lanes 2 to 19 and 22 to 35: Total nucleic acids isolated from whole blood samples obtained from cattle with BSE. Each lane represents a sample taken from an single animal. In the upper part of each lane genomic DNA is visible, the two distinct lower bands correspond to 28S and 18S ribosomal RNAs. Lane 20 and 36: Isolate from HeLa cells (control). The gel was stained using SYBR Green Gel Stain (Molecular Probes).

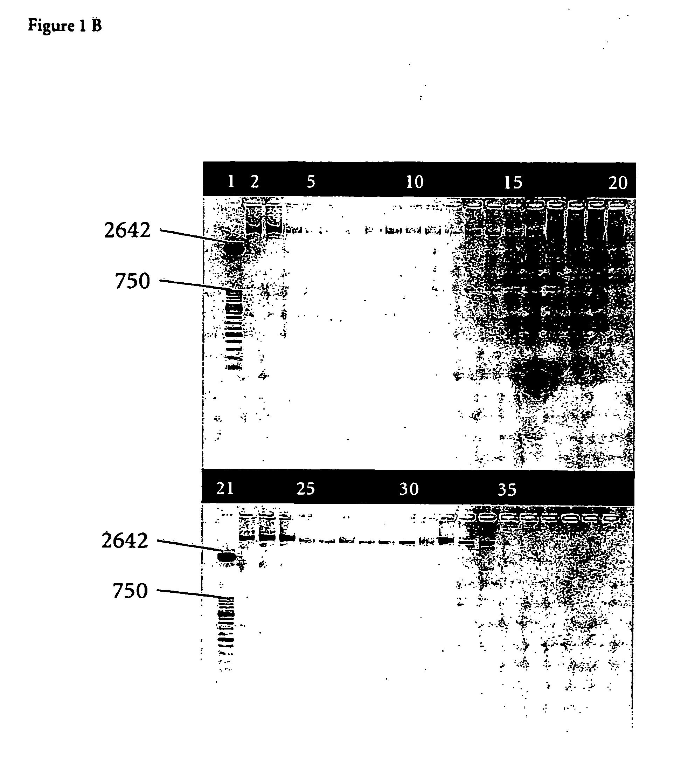

- **Figure 1B**: Lanes 1 and 21: Size marker, fragment sizes in base pairs are indicated on the left. Lanes 2 to 13: Total nucleic acids isolated from animals which were BSE suspects but were tested negative post mortem. Lanes 14 to 34: Total nucleic acids isolated from whole blood samples obtained from BSE-negative cattle. Each lane represents a sample taken from an single animal. In the upper part of each lane genomic DNA is visible, the two distinct lower bands correspond to 28S and 18S ribosomal RNAs. The gel was stained using SYBR^{®} Green Gel Stain (Molecular Probes).

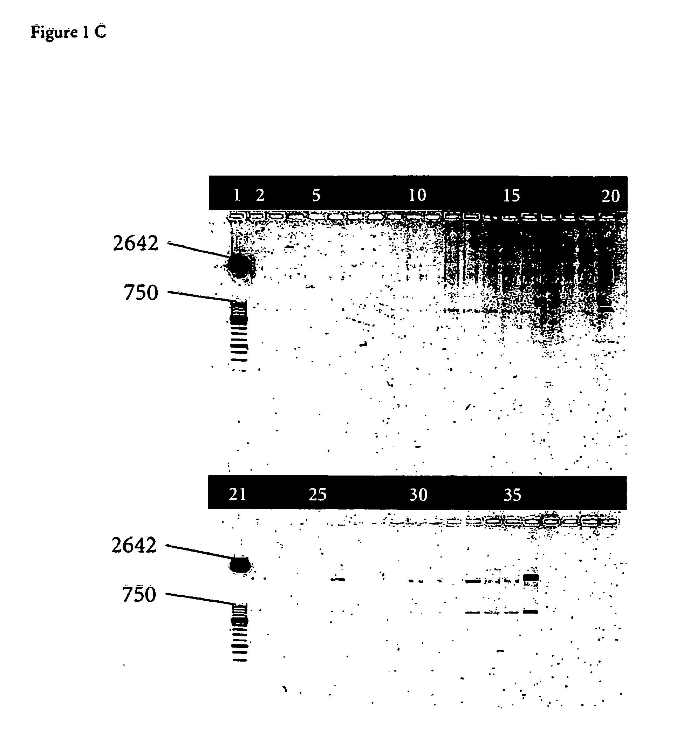

- **Figure 1C**: Lanes 1 and 21: Size marker, fragment sizes in base pairs are indicated on the left. Lanes 2 to 19 and lanes 22 to 35: Total RNA isolated from whole blood samples obtained from cattle with BSE. Each lane represents a sample taken from a single animal. Distinct bands correspond to 28S and 18S ribosomal RNAs. Lane 20 and 36: Isolate from HeLa cells (control). The gel was stained using SYBR Green Gel Stain (Molecular Probes).

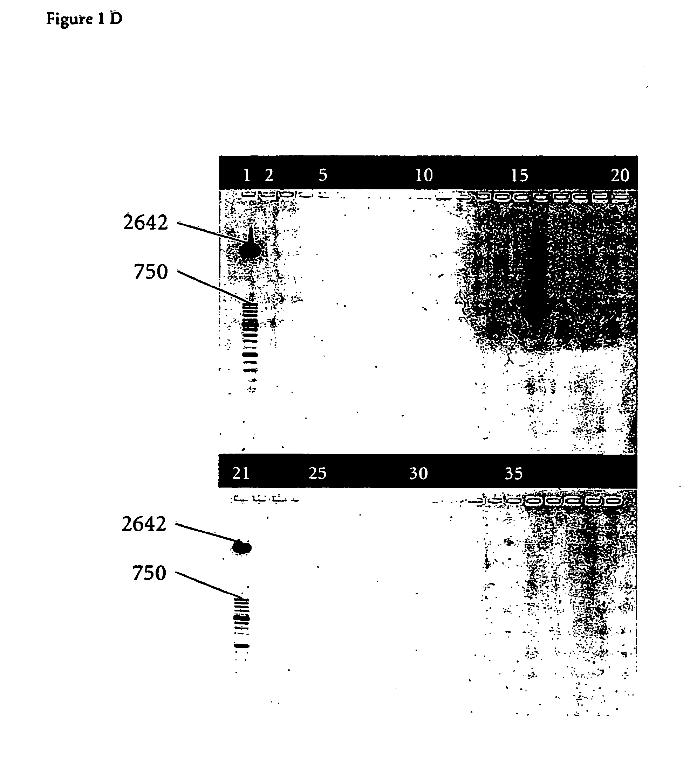

- **Figure 1D**: Lanes 1 and 21: Size marker, fragment sizes in base pairs are indicated on the left. Lanes 2 to 13: Total RNA isolated from animals which were BSE suspects but were tested negative post mortem. Lanes 14 to 34: Total RNA isolated from whole blood samples obtained from BSE-negative cattle. Each lane represents a sample taken from an single animal. Distinct bands correspond to 28S and 18S ribosomal RNAs. The gel was stained using SYBR Green Gel Stain (Molecular Probes).
- **Figure 2**: Agarose gel showing the results of RT-PCR from whole blood samples obtained from a set of 16 BSE-infected or PrP^{sc}-positive cattle and 10 cattle with a negative (PrP^{sc}-negative) result of a post-mortem test on brain stem tissue. The white arrows identify PCR fragments which have been sequenced.
A further description of the samples is given in Example 1.
Lane 1: DNA Molecular Weight Marker XIII (50-750 bp), Roche Diagnostics (catalogue no. 1 721 925). Mixture of restriction fragments from a plasmid; fragment sizes are 50, 100, 150, 200, 250, 500, 750 (black arrow), 2642 bp (black arrow).
Lane 2: Control plasmid, there are three bands (amplification products) in the gel due to the high concentration of plasmid DNA in the PCR reaction mixture.
Lane 3: water control (negative control)
Lane 4 to Lane 13: A1 samples
Lane 14: A2-g
Lane 15: A2-h
Lane 16: A2-a
Lane 17: A2-b
Lane 18: A2-c
Lane 19: A2-d
Lane 20: C1-a
Lane 21: DNA Molecular Weight Marker XIII (50-750 bp), Roche Diagnostics (catalogue no. 1 721 925). Mixture of restriction fragments from a plasmid; fragment sizes are 50, 100, 150, 200, 250, 500, 750 (black arrow), 2642 bp (black arrow).
Lane 22: C1-b
Lane 23: C1-c
Lane 24: C1-d
Lane 25: Cl-e
Lane 26: C1-f
Lane 27 and
Lane 28: B2
Lane 29 and
Lane 30: B1
Lane 31: A1 sample spiked with control plasmid (10⁶ dilution)
Lane 32: A1 sample spiked with control plasmid (10⁹ dilution)
Lane 33: C1 sample spiked with control plasmid (10⁶ dilution)
Lane 34: C1 sample spiked with control plasmid (10⁹ dilution)
- **Figure 3**: Alignment of amino acid sequences of human cathepsin K precursor (AAX09069 (SEQ ID NO: 18), P43235 (SEQ ID NO: 20)), bovine cathepsin K precursor (BT021052, SEQ ID NO: 19), human cathepsin O precursor (P43234 (SEQ ID NO: 22), NP_001325 (SEQ ID NO: 21)), bovine cathepsin K precursor (BT021052), murine cathepsin O precursor (Q8BM88, SEQ ID NO: 23), SEQ ID NO:2 and the conceptual catalytic center of the hypothetical cystein protease (Cys motif, SEQ ID NO: 24). The asterisk marks the position of the cysteine residue which is a hallmark of the catalytic center in cysteine proteases.

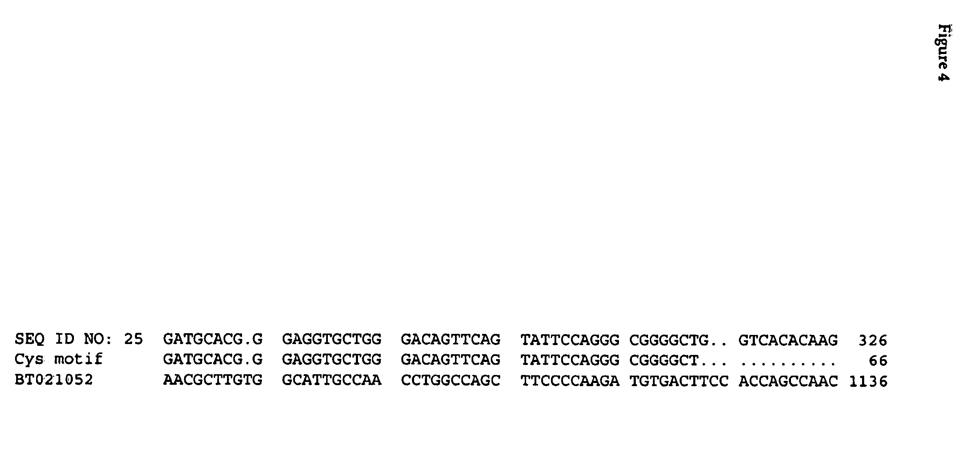

- **Figure 4**: Nucleotide sequence alignment comparing the nucleotide sequence encoding the Cys motif (SEQ ID NO: 26) with the corresponding sequence encoding the bovine cathepsin K precursor (BT021052) (SEQ ID NO: 27). The coding strands of the two sequences were aligned with the base triplett encoding the characteristic cysteine residue in the active center of of cysteine proteases. The top line of the sequence alignment is disclosed as SEQ ID NO:25.

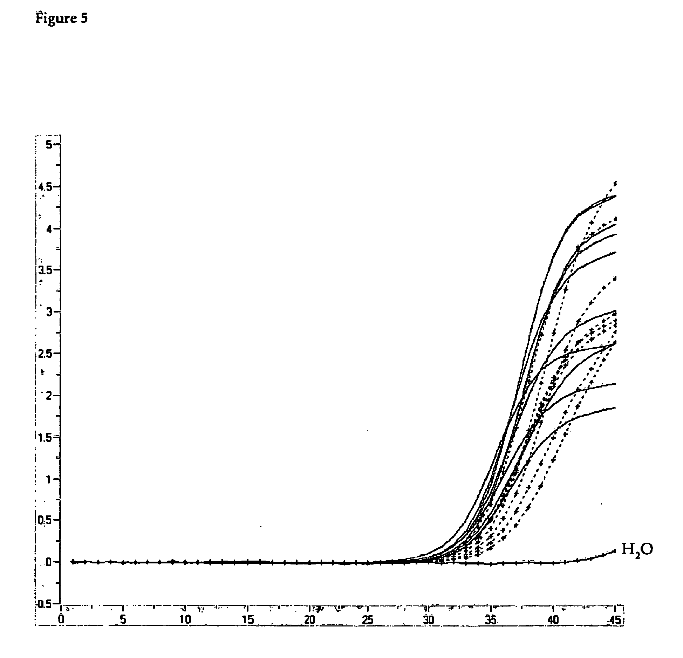

- **Figure 5**: Results from real-time RT-PCR of total RNA from whole blood samples of cattle using a LightCycler^{®} instrument and the experimental setting as described in Example 4. Solid lines indicate individual PCR experiments with samples from BSE-infected animals, dotted lines indicate BSE-free animals. The Figure further illustrates the crossing point data given in Table 2. The ordinate indicates the values for fluorescence (F2/Back-F1) as determined by the LightCycler^{®} instrument, the abscissa indicates cycle numbers. The water control is marked by "H₂O".

### Example 1

### Blood samples (cattle)

A first set of samples from cattle, designated "A1", comprised 10 individuals which suffered from a natural infection with the BSE inducing infective agent (field cases). By way of immunological detection of PrP^{sc} in brainstem (obex) tissue with a officially approved post mortem test a positive test result was established for each A1 animal.

A further set of blood samples, designated "A2", was obtained from experimentally inoculated animals. These animals were infected by feeding with brain homogenate (100 g or 1 g) obtained from cattle which were naturally infected with BSE. Two samples ("A2-g" and "A2-h") were from inoculated animals without any sign of BSE. Two samples ("A2-a" and "A2-b") were from animals which showed definite signs of BSE. Three samples ("A2-c" and "A2-d") were from animals which showed possible signs of BSE.

Two healthy animals of the negative (i.e. not infected) control group (designated "B2") were included in the analysis.

A further set of samples (designated "B1") was obtained from healthy animals (beef cattle) prior to slaughtering. All B1 animals were tested for BSE by way of immunological detection of PrP^{sc} in brainstem using an officially approved post mortem test.

Another set of blood samples derived from cattle which were suspected of being infected with BSE (group designation "C1"). All C1 animals were tested for BSE by way of immunological detection of PrP^{sc} in brainstem using an officially approved post mortem test. With regard to clinical signs of BSE, one animal ("C1=a") was inconclusive, another ("C1-d") showed definite signs of BSE. Four other C1 samples came from animals without clinical signs of BSE.

### Example 2

### Nucleic acid preparation

RNA extraction from whole blood samples was performed using the MagNA Pure^{®} LC intrument and either (a) the MagNA Pure^{®} LC mRNA isolation kit 1 for blood and blood cells (Roche Diagnostics GmbH, Mannheim, Applied Science catalogue number 03 004 015), (b) the MagNA Pure^{®} LC RNA isolation kit High Performance (Roche Diagnostics GmbH, Mannheim, Applied Science catalogue number 03 542 394), (c) the MagNA Pure^{®} LC total NA isolation kit - large volume (Roche Diagnostics GmbH, Mannheim, Applied Science catalogue number 03 264 793) or (d) MagNA Pure^{®} total NA Isolation kit (Roche Diagnostics GmbH, Mannheim, Applied Science catalogue number 03 038 505). RNA and total NA were isolated according to the instructions of the manufacturer.

Following purification an aliquot of 5 µl of each preparation (corresponding to 5% of the respective total preparation) was electrophoresed on a 0.8% agarose gel and stained with SYBR Green^{®} I. Figures 1 A, B, C, and D shows exemplary agarose electrophoresis gels.

### Example 3

### Pre-screening for BSE-specific marker RNAs and analysis of putative marker sequences

Light Cycler^{®} RT-PCR was performed using the LC Fast Start DNA Master^{Plus} SYBR Green^{®} I kit (Roche Diagnostics GmbH, Mannheim, Applied Science catalogue no. 03 515 869) and a LightCycler^{®} 1.2 instrument according to the instructions of the manufacturer.

A first round of screening attempted the detection of BSE-specific RNA sequences in whole blood samples of BSE-infected and not infected cattle. Amplified sequences were characterized and optimized primers were designed and tested. Among a larger collection of primer pairs tested the two oligonucleotides LTR895for and LTR895rev according to SEQ ID NOs: 3 and 4 were characterized in first RT-PCR experiments.

In order to provide positive controls, the target sequences for the two primers were cloned in a plasmid vector ("control plasmid") in an arrangement such that PCR of the control plasmid yielded an amplified fragment of the plasmid with a size of 350 bp. In control experiments, a solution with a measured amount of isolated control plasmid DNA (Table 1) was used.

**Table 1:**

| **Control plasmid for LTR895 primers, purity and concentration in aequous solution** | | | | |
|---|---|---|---|---|
| 260 nm | 280 nm | 320 nm | ratio_{260/280} | concentration, in µg/ml |
| 0.062 | 0.034 | 0.001 | 1.823 | 61 |

Extinctions at indicated wavelengths were determined using a NanoDrop ND 1000 UV/Vis spectrophotometer. The conditions for PCR were adjusted using the control plasmid, in order to optimize primer concentration and annealing temperature. In addition, amplification was tested at different concentrations of the control plasmid.

Whole blood RNA preparations from a set of 16 BSE-infected and 10 not infected cattle were then subjected to RT-PCR analysis using the LTR895 primer pair. Resulting amplified DNA fragments were electrophoresed in agarose gels and selected bands of the electrophoretic banding pattern were analyzed. Figure 2 shows a banding pattern obtained from testing 16 BSE-infected and 10 not infected cattle, together with positive and negative controls.

Four PCR fragments migrating at about 400 bp were isolated and sequenced. Each yielded the nucleotide sequence given in SEQ ID NO: 1. The observation that this fragment turned up in three infected and one clinical BSE suspect case (post-mortem BSE negative) induced further experiments in order to evaluate the diagnostic potential of the target RNA sequence.

### Example 4

### Validation

To prove the potential of the 369bp RNA fragment according to SEQ ID NO:1 to discriminate between BSE positive (post-mortem) and healthy cattle primers within SEQ ID NO:1 were designed for amplification of subfragments. Preferred forward primers were the primers accorsing to SEQ ID NOs:5, 7, 9, 11, and 13. Preferred reverse primers were the primers accorsing to SEQ ID NOs:6, 8, 10, 12, 14, 15, and 16. The primer design results in PCR products which are shorter than SEQ ID NO:1.

PCR was performed as RT-PCR in a Light Cycler^{®} 1.2 instrument using total RNA isolated from whole blood samples and the LC Fast Start DNA Master^{Plus} SYBR Green^{®} I kit (Roche Diagnostics GmbH, Mannheim, Applied Science catalogue no. 03 515 869).

For the respective PCR experiments the primers LTR895(typeI)-1.for and LTR895(typeI)-1.rev according to SEQ ID NO: 5 and SEQ ID NO: 6 were used with particular preference. Real-time PCR analytics with the Roche LightCycler^{®} 1.2 instrument revealed discrimination of (i) diseased BSE-infected and (ii) healthy animals: BSE animals which were tested as positives post mortem showed a crossing point of 33.2 (mean value) compared to a crossing point of 34.9 (mean value) obtained for healthy individuals (Table 2).

In a PCR amplification reaction, the cycle at which the fluorescence, i.e. in the present case the fluorescence of the complex comprising SYBR^{®} Green and the amplified DNA, rises above the background fluorescence is called the "crossing point" of the sample. The crossing point of a sample appears as a sharp upward curve on the experiment's fluorescence chart. The crossing point is the point at which the amplified product is first visible in the data. A sample's crossing point depends on the initial concentration of the target nucleic acid in the sample.

A sample with a lower initial concentration of the target nucleic acid requires more amplification cycles to reach the crossing point. To this end, prior to PCR analysis normalization of nucleic acids was performed to ensure equal RNA amounts in each PCR setup. For nucleic acid normalization the nucleic acid yield of all samples to be compared in a real time PCR run was determined by measuring the OD₂₆₀ₙₘ. Depending on the resulting nucleic acid yield all samples were diluted to result in identical nucleic acid concentrations. By way of normalization the real-time PCR results of different samples became comparable.

Crossing points determined for individual samples are presented in Table 2. The fluorescence chart of the RT-PCR experiments is given as Figure 5.

**Table 2:**

| **Crossing points determined in real-time PCR experiments using the LightCycler^{®} instrument** | | |
|---|---|---|
| Sample | Status | Crossing point |
| H₂O | negative run control | > 41.00 |
| 1 | infected, clinical signs of BSE, confirmed by post-mortem test | 32.49 |
| 2 | infected, clinical signs of BSE, confirmed by post-mortem test | 32.93 |
| 3 | infected, clinical signs of BSE, confirmed by post-mortem test | 33.94 |
| 4 | infected, clinical signs of BSE, confirmed by post-mortem test | 33.53 |
| 5 | infected, clinical signs of BSE, confirmed by post-mortem test | 33.94 |
| 6 | infected, clinical signs of BSE, confirmed by post-mortem test | 33.76 |
| 7 | infected, clinical signs of BSE, confirmed by post-mortem test | 31.91 |
| 8 | infected, clinical signs of BSE, confirmed by post-mortem test | 33.04 |
| 9 | infected, clinical signs of BSE, confirmed by post-mortem test | 33.12 |
| 10 | infected, clinical signs of BSE, confirmed by post-mortem test | 33.78 |
| **BSE, average** | | **33.2** |
| 11 | healthy, not infected, negative post-mortem test | 35.46 |
| 12 | healthy, not infected, negative post-mortem test | 35.52 |
| 13 | healthy, not infected, negative post-mortem test | 34.07 |
| 14 | healthy, not infected, negative post-mortem test | 34.06 |
| 15 | healthy, not infected, negative post-mortem test | 36.50 |
| 16 | healthy, not infected, negative post-mortem test | 34.04 |
| 17 | healthy, not infected, negative post-mortem test | 35.50 |
| 18 | healthy, not infected, negative post-mortem test | 34.19 |
| **healthy, average** | | **34.9** |

## Claims

1. Use of a nucleotide sequence encoding a fragment of a hypothetical cystein proteinase according to SEQ ID NO:2 or a nucleotide sequence complementary thereto, as a marker in the assessment of transmissible spongiform encephalopathy (TSE) in a bovine.

2. Use according to claim 1, **characterized in that** the nucleotide sequence encoding the fragment of a hypothetical cystein proteinase is SEQ ID NO:1.

3. Method for assessing transmissible spongiform encephalopathy (TSE) in a bovine by detecting a target RNA, comprising the consecutive steps, in a sample obtained from the bovine, of
(a) preparing RNA from the sample,
(b) detecting the target RNA in the RNA preparation of step (a),
(c) using the result of step (b) to assess TSE in the bovine,
**characterized in that**
the sample from the bovine is whole blood, and the target RNA encodes an amino acid sequence according to SEQ ID NO:2 or a subfragment thereof comprising 6 or more consecutive amino acids.

4. The method according to claim 3, comprising the steps, in a whole blood sample obtained from the bovine, of
(a) preparing RNA from the sample
(b) providing a pair of primers comprising a first and a second primer,
whereby the first primer consists of 12 or more contiguous nucleotides of a nucleic acid sequence encoding the amino acid sequence according to SEQ ID NO:2, and
whereby the second primer consists of 12 or more contiguous nucleotides of a nucleic acid sequence complementary to the nucleic acid sequence encoding the amino acid sequence according to SEQ ID NO:2;
(c) amplifying a target nucleic acid from the RNA preparation of step (a) with the primers of step (b);
(d) detecting the target nucleic acid of step (c);
(e) subsequently using the result of the detection of step (d) to assess TSE in the bovine.

5. The method according to claim 4, **characterized in that** the target nucleic acid is amplified by way of polymerase chain reaction.

6. The method according to any of the claims 4 and 5, **characterized in that** the amplified target nucleic acid is detected using a fluorescent signal.

7. Method according to claim 6, **characterized in that** the target nucleic acid is detected by monitoring the amplification in real time and determining the amount of amplification product after each cycle.

8. Method according to any of the claims 4 to 7, **characterized in that** the target nucleic acid is the nucleotide sequence according to SEQ ID NO:1 or a subfragment thereof.

9. The method according to any of the claims 4 to 8, **characterized in that** the first primer is the nucleotide sequence according to SEQ ID NO:5, and the second primer is the nucleotide sequence according to SEQ ID NO:6.

10. A kit of parts for assessing TSE in a mammal by detecting a target RNA, comprising (i) two oligonucleotide primers for amplification of a target nucleic acid, whereby the first primer is a nucleic acid sequence selected from the group consisting of SEQ ID NO:5, SEQ ID NO:7, SEQ ID NO:9, SEQ ID NO:11, and SEQ ID NO:13, and whereby the second primer is a nucleic acid sequence selected from the group consisting of SEQ ID NO:6, SEQ ID NO:8, SEQ ID NO:10, SEQ ID NO:12, SEQ ID NO:14. SEQ ID NO:15, and SEQ ID NO:16. and (ii) an intercalating dye.

11. A kit of parts for assessing TSE in a mammal by detecting a target RNA, comprising (i) two oligonucleotide primers for amplification of a target nucleic acid, whereby the first primer is a nucleic acid sequence selected from the group consisting of SEQ ID NO:5, SEQ ID NO:7, SEQ ID NO:9, SEQ ID NO:11, and SEQ ID NO:13, and whereby the second primer is a nucleic acid sequence selected from the group consisting of SEQ ID NO:6, SEQ ID NO:8, SEQ ID NO:10, SEQ ID NO:12, SEQ ID NO:14, SEQ ID NO:15, and SEQ ID NO:16, and (ii) a first and a second oligonucleotide hybridization probe labeled with a first and a second fluorescent moiety, respectively, whereby the two oligonucleotide hybridization probes are complementary to adjacent but non overlapping regions of one strand of the target nucleic acid, whereby the first oligonucleotide hybridization probe is labeled at the 5' end and the second oligonucleotide hybridization probe is labeled at the 3' end, and whereby the first fluorescent labels are capable of effecting fluorescence resonance energy transfer.

12. A kit of parts for assessing TSE in a mammal by detecting a target RNA, comprising (i) two oligonucleotide primers for amplification of a target nucleic acid, whereby the first primer is a nucleic acid sequence selected from the group consisting of SEQ ID NO:5, SEQ ID NO:7, SEQ ID NO:9. SEQ ID NO:11, and SEQ ID NO:13. and whereby the second primer is a nucleic acid sequence selected from the group consisting of SEQ ID NO:6, SEQ ID NO:8, SEQ ID NO:10, SEQ ID NO:12, SEQ ID NO:14, SEQ ID NO:15, and SEQ ID NO:16, and (ii) a single-stranded hybridization probe which is labeled with a fluorescent entity, the fluorescence emission of which is quenched by a second label on the same probe, whereby the single-stranded hybridization probe is complementary to a region of one strand of the target nucleic acid.

13. A kit of parts for assessing TSE in a mammal by detecting a target RNA, comprising (i) two oligonucleotide primers for amplification of a target nucleic acid, whereby the first primer is a nucleic acid sequence selected from the group consisting of SEQ ID NO:5, SEQ ID NO:7, SEQ ID NO:9, SEQ ID NO:11, and SEQ ID NO:13, and whereby the second primer is a nucleic acid sequence selected from the group consisting of SEQ ID NO:6, SEQ ID NO:8, SEQ ID NO:10, SEQ ID NO:12, SEQ ID NO:14, SEQ ID NO:15, and SEQ ID NO:16, and (ii) a molecular beacon oligonucleotide complementary to a region of one strand of the target nucleic acid, whereby the molecular beacon oligonucleotide is labeled at opposite ends with a fluorescent compound and a quencher compound, which due to the secondary structure of said molecular beacon oligonucleotide are in close vicinity to each other.

## Patentansprüche

1. Verwendung einer Nukleotidsequenz, die für ein Fragment einer hypothetischen Cysteinproteinase gemäß SEQ ID NO:2 oder für eine dazu komplementäre Nukleotidsequenz codiert, als Marker bei der Beurteilung von übertragbarer spongiformer Enzephalopathie (TSE) bei einem Rind.

2. Verwendung nach Anspruch 1, **dadurch gekennzeichnet, daß** es sich bei der Nukleotidsequenz, die für das Fragment einer hypothetischen Cysteinproteinase codiert, um SEQ ID NO:1 handelt.

3. Verfahren zur Beurteilung der übertragbaren spongiformen Enzephalopathie (TSE) bei einem Rind durch Nachweisen einer Ziel-RNA, umfassend die folgenden nacheinander an einer vom Rind stammenden Probe durchgeführten Schritte:
(a) Präparieren der RNA aus der Probe,
(b) Nachweisen der Ziel-RNA in dem RNA-Präparat von Schritt (a),
(c) Beurteilen von TSE in dem Rind aufgrund des Ergebnisses von Schritt (b),
**dadurch gekennzeichnet, daß**
es sich bei der Probe aus dem Rind um Vollblut handelt und daß die Ziel-RNA für eine Aminosäuresequenz gemäß SEQ ID NO:2 oder ein Teilfragment davon mit 6 oder mehr aufeinanderfolgenden Aminosäuren codiert.

4. Verfahren nach Anspruch 3, umfassend folgenden die an einer vom Rind stammenden Vollblutprobe durchgeführten Schritte:
(a) Präparieren von RNA aus der Probe,
(b) Bereitstellen eines Primer-Paars, das einen ersten und einen zweiten Primer umfaßt, wobei der erste Primer aus 12 oder mehr unmittelbar aufeinanderfolgenden Nukleotiden einer Nukleinsäuresequenz, die für die Aminosäuresequenz gemäß SEQ ID NO:2 codiert, besteht und
wobei der zweite Primer aus 12 oder mehr unmittelbar aufeinanderfolgenden Nukleotiden einer Nukleinsäuresequenz, die zu der Nukleinsäuresequenz, die für die Aminosäuresequenz gemäß SEQ ID NO:2 codiert, komplementär ist, besteht;
(c) Amplifizieren einer Zielnukleinsäure aus dem RNA-Präparat von Schritt (a) mit den Primern von Schritt (b);
(d) Nachweisen der Zielnukleinsäure von Schritt (c) ;
(e) anschließendes Beurteilen von TSE bei dem Rind aufgrund des Ergebnisses des Nachweises von Schritt (d)

5. Verfahren nach Anspruch 4, **dadurch gekennzeichnet, daß** die Zielnukleinsäure mittels Polymerasekettenreaktion amplifiziert wird.

6. Verfahren nach einem der Ansprüche 4 und 5, **dadurch gekennzeichnet, daß** die amplifizierte Zielnukleinsäure mit einem Fluoreszenzsignal nachgewiesen wird.

7. Verfahren nach Anspruch 6, **dadurch gekennzeichnet, daß** die Zielnukleinsäure durch Echtzeit-Verfolgung der Amplifikation und Bestimmen der Amplifikationsproduktmenge nach jedem Zyklus nachgewiesen wird.

8. Verfahren nach einem der Ansprüche 4-7, **dadurch gekennzeichnet, daß** es sich bei der Zielnukleinsäure um die Nukleotidsequenz gemäß SEQ ID NO:1 oder ein Teilfragment davon handelt.

9. Verfahren nach einem der Ansprüche 4-8, **dadurch gekennzeichnet, daß** es sich bei dem ersten Primer um die Nukleotidsequenz gemäß SEQ ID NO:5 und bei dem zweiten Primer um die Nukleotidsequenz gemäß SEQ ID NO:6 handelt.

10. Aus Teilen bestehendes Kit für die Beurteilung von TSE bei einem Säugetier durch Nachweisen einer Ziel-RNA, umfassend (i) zwei Oligonukleotid-Primer für die Amplifikation einer Zielnukleinsäure, wobei es sich bei dem ersten Primer um eine Nukleinsäuresequenz aus der Gruppe SEQ ID NO:5, SEQ ID NO:7, SEQ ID NO:9, SEQ ID NO:11 und SEQ ID NO:13 handelt und wobei es sich bei dem zweiten Primer um eine Nukleinsäuresequenz aus der Gruppe SEQ ID NO:6, SEQ ID NO:8, SEQ ID NO:10, SEQ ID NO:12, SEQ ID NO:14, SEQ ID NO:15 und SEQ ID NO:16 handelt, sowie (ii) einen interkalierenden Farbstoff.

11. Aus Teilen bestehendes Kit für die Beurteilung von TSE bei einem Säugetier durch Nachweisen einer Ziel-RNA, umfassend (i) zwei Oligonukleotid-Primer für die Amplifikation einer Zielnukleinsäure, wobei es sich bei dem ersten Primer um eine Nukleinsäuresequenz aus der Gruppe SEQ ID NO:5, SEQ ID NO:7, SEQ ID NO:9, SEQ ID NO:11 und SEQ ID NO:13 handelt und wobei es sich bei dem zweiten Primer um eine Nukleinsäuresequenz aus der Gruppe SEQ ID NO:6, SEQ ID NO:8, SEQ ID NO:10, SEQ ID NO:12, SEQ ID NO:14, SEQ ID NO:15 und SEQ ID NO:16 handelt, sowie (ii) eine erste und eine zweite Oligonukleotidhybridisierungssonde, die mit einer ersten bzw. einer zweiten Fluoreszenzgruppe markiert ist, wobei die beiden Oligonukleotidhybridisierungssonden zu nebeneinander liegenden, jedoch nicht überlappenden, Regionen eines Strangs der Zielnukleinsäure komplementär sind, wobei die erste Oligonukleotidhybridisierungssonde am 5'-Ende und die zweite Oligonukleotidhybridisierungssonde am 3'-Ende markiert ist, und wobei die ersten Fluoreszenzmarker fähig sind, einen Fluoreszenzresonanzenergietransfer durchzuführen.

12. Aus Teilen bestehendes Kit für die Beurteilung von TSE bei einem Säugetier durch Nachweisen einer Ziel-RNA, umfassend (i) zwei Oligonukleotid-Primer für die Amplifikation einer Zielnukleinsäure, wobei es sich bei dem ersten Primer um eine Nukleinsäuresequenz aus der Gruppe SEQ ID NO:5, SEQ ID NO:7, SEQ ID NO:9, SEQ ID NO:11 und SEQ ID NO:13 handelt und wobei es sich bei dem zweiten Primer um eine Nukleinsäuresequenz aus der Gruppe SEQ ID NO:6, SEQ ID NO:8, SEQ ID NO:10, SEQ ID NO:12, SEQ ID NO:14, SEQ ID NO:15 und SEQ ID NO:16 handelt, sowie (ii) eine einzelsträngige Hybridisierungssonde, die mit einem Fluoreszenzteil markiert ist, dessen Fluoreszenzemission durch einen zweiten Marker an derselben Sonde gequencht wird, wobei die einzelsträngige Hybridisierungssonde zu einer Region eines Strangs der Zielnukleinsäure komplementär ist.

13. Aus Teilen bestehendes Kit für die Beurteilung von TSE bei einem Säugetier durch Nachweisen einer Ziel-RNA, umfassend (i) zwei Oligonukleotid-Primer für die Amplifikation einer Zielnukleinsäure, wobei es sich bei dem ersten Primer um eine Nukleinsäuresequenz aus der Gruppe SEQ ID NO:5, SEQ ID NO:7, SEQ ID:NO:9, SEQ ID NO:11 und SEQ ID NO:13 handelt und wobei es sich bei dem zweiten Primer um eine Nukleinsäuresequenz aus der Gruppe SEQ ID NO:6, SEQ ID NO:8, SEQ ID NO:10, SEQ ID NO:12, SEQ ID NO:14, SEQ ID NO:15 und SEQ ID NO:16 handelt, sowie (ii) ein Molecular-Beacon-Oligonukleotid, das zu einer Region eines Strangs der Zielnukleinsäure komplementär ist, wobei das Molecular-Beacon-Oligonukleotid an entgegengesetzten Enden mit einer fluoreszierenden Verbindung und einer Quencher-Verbindung, die aufgrund der Sekundärstruktur des Molekular-Beacon-Oligonukleotids eng benachbart sind, markiert ist.

## Revendications

1. Utilisation d'une séquence nucléotidique codant pour un fragment d'une cystéine protéinase hypothétique selon SEQ ID NO :2 ou une séquence nucléotidique complémentaire à celle-ci, en tant que marqueur dans l'évaluation d'une encéphalopathie spongiforme transmissible (TSE) chez un bovin.

2. Utilisation selon la revendication 1, **caractérisée en ce que**, la séquence nucléotidique codant pour le fragment d'une cystéine protéinase hypothétique est SEQ ID NO :1.

3. Procédé pour évaluer l'encéphalopathie spongiforme transmissible (TSE) chez un bovin en détectant un ARN cible, comprenant les étapes consécutives,
dans un échantillon obtenu chez le bovin, de
(a) préparation de l'ARN à partir de l'échantillon,
(b) détection de l'ARN cible dans la préparation d'ARN de l'étape (a),
(c) utilisation du résultat de l'étape (b) pour évaluer TSE chez le bovin,
**caractérisé en ce que**,
l'échantillon provenant du bovin est du sang entier, et l'ARN cible code pour une séquence d'acides aminés selon SEQ ID NO :2 ou un sous-fragment de celle-ci comprenant 6 acides aminés consécutifs ou plus.

4. Procédé selon la revendication 3, comprenant les étapes dans un échantillon de sang entier obtenu à partir du bovin, de
(a) préparation de l'ARN à partir de l'échantillon
(b) fourniture d'une paire d'amorces comprenant une première et une deuxième amorce,
où la première amorce est constituée de 12 nucléotides contigus ou plus d'une séquence d'acide nucléique codant pour la séquence d'acides aminés selon SEQ ID NO :2, et
où la deuxième amorce est constituée de 12 nucléotides contigus ou plus d'une séquence d'acide nucléique complémentaire à la séquence d'acide nucléique codant pour la séquence d'acides aminés selon SEQ ID NO :2 ;
(c) amplification d'un acide nucléique cible à partir de la préparation d'ARN de l'étape (a) avec les amorces de l'étape (b) ;
(d) détection de l'acide nucléique cible de l'étape (c) ;
(e) utilisation ultérieure du résultat de la détection de l'étape (d) pour évaluer TSE chez le bovin.

5. Procédé selon la revendication 4, **caractérisé en ce** l'acide nucléique cible est amplifié au moyen d'une amplification en chaîne par polymérase.

6. Procédé selon l'une quelconque des revendications 4 et 5, **caractérisé en ce que** l'acide nucléique cible amplifié est détecté en employant un signal fluorescent.

7. Procédé s elon la revendication 6, **caractérisé** e n ce que, l'acide nucléique cible est détecté en contrôlant l'amplification en temps réel et en déterminant la quantité de produit d'amplification après chaque cycle.

8. Procédé selon l'une quelconque des revendications 4 à 7, **caractérisé en ce que** l'acide nucléique cible est la séquence nucléotidique selon SEQ ID NO :1 ou un sous-fragment de celle-ci.

9. Procédé selon l'une quelconque des revendications 4 à 8, **caractérisé en ce que**, la première amorce est la séquence nucléotidique selon SEQ ID NO :5, et la deuxième amorce est la séquence nucléotidique selon SEQ ID NO :6.

10. Nécessaire de pièces pour évaluer TSE chez un mammifère en détectant un ARN cible, comprenant (i) deux amorces oligonucléotidiques pour l'amplification d'un acide nucléique cible, où la première amorce est une séquence d'acide nucléique choisie dans le groupe formé par SEQ ID NO :5, SEQ ID NO :7, SEQ ID NO :9, SEQ ID NO :11, et SEQ ID NO :13, et où la deuxième amorce est une séquence d'acide nucléique choisie dans le groupe formé par SEQ ID NO :6, SEQ ID NO :8, SEQ ID NO :10, SEQ ID NO :12, SEQ ID NO :14, SEQ ID NO :15, et SEQ ID NO :16, et (ii) un colorant intercalaire.

11. Nécessaire de pièces pour évaluer TSE chez un mammifère en détectant un ARN cible, comprenant (i) deux amorces oligonucléotidiques pour l'amplification d'un acide nucléique cible, où la première amorce est une séquence d'acide nucléique choisie dans le groupe formé par SEQ ID NO :5, SEQ ID NO :7, SEQ ID NO :9, SEQ ID NO :11, et SEQ ID NO :13, et où la deuxième amorce est une séquence d'acide nucléique choisie dans le groupe formé par SEQ ID NO :6, SEQ ID NO :8, SEQ ID NO :10, SEQ ID NO :12, SEQ ID NO :14, SEQ ID NO :15, et SEQ ID NO:16, et (ii) une première et une deuxième sonde d'hybridation oligonucléotidiques, respectivement marquées avec une première et une deuxième fraction fluorescente, où les deux sondes d'hybridation oligonucléotidiques sont complémentaires aux régions adjacentes mais qui ne se chevauchent pas, d'un brin de l'acide nucléique cible, où la première sonde d'hybridation oligonucléotidique est marquée à l'extrémité 5' et où la deuxième sonde d'hybridation oligonucléotidique est marquée à l'extrémité 3' et où les premiers marqueurs fluorescents sont capables de réaliser un transfert d'énergie par résonance de fluorescence.

12. Nécessaire de pièces pour évaluer TSE chez un mammifère, en détectant un ARN cible, comprenant (i) deux amorces oligonucléotidiques pour l'amplification d'un acide nucléique cible, où la première amorce est une séquence d'acide nucléique choisie dans le groupe formé par SEQ ID NO :5, SEQ ID NO :7, SEQ ID NO :9, SEQ ID NO :11, et SEQ ID NO :13, et où la deuxième amorce est une séquence d'acide nucléique choisie dans le groupe formé par SEQ ID NO :6, SEQ ID NO :8, SEQ ID NO :10, SEQ ID NO :12, SEQ ID NO:14, SEQ ID NO :15, et SEQ ID NO:16, et (ii) une sonde d'hybridation à simple brin qui est marquée par une entité fluorescente, dont l'émission de fluorescence est éteinte par un deuxième marquage sur la même sonde, où la sonde d'hybridation à simple brin est complémentaire à une région d'un brin de l'acide nucléique cible.

13. Nécessaire de pièces pour évaluer TSE chez un mammifère, en détectant un ARN cible, comprenant (i) deux amorces oligonucléotidiques pour l'amplification d'un acide nucléique cible, où la première amorce est une séquence d'acide nucléique choisie dans le groupe formé par SEQ ID NO :5, SEQ ID NO :7, SEQ ID NO :9, SEQ ID NO :11, et SEQ ID NO :13, et où la deuxième amorce est une séquence d'acide nucléique choisie dans le groupe formé par SEQ ID NO :6, SEQ ID NO :8, SEQ ID NO :10, SEQ ID NO :12, SEQ ID NO :14, SEQ ID NO :15, et SEQ ID NO :16, et (ii) un oligonucléotide de balise moléculaire complémentaire à une région d'un brin de l'acide nucléique cible, où l'oligonucléotide de balise moléculaire est marqué aux extrémités opposées par un composé fluorescent et un composé extincteur, qui, en raison de la structure secondaire dudit oligonucléotide de balise moléculaire, sont au voisinage immédiat l'un de l'autre.
